# EUROPÄISCHE PATENTSCHRIFT

(11) **EP 1 763 522 B1**
(45) Veröffentlichungstag und Bekanntmachung des Hinweises auf die Patenterteilung: **20.03.2013**
(21) Anmeldenummer: 05761787.0
(22) Anmeldetag: 03.06.2005
(51) Int. Cl.: C07D 401/12, A61K 31/506, A61P 25/16, A61P 25/18, A61P 25/24

(54) **PYRIDIN-2-ONVERBINDUNGEN UND DEREN VERWENDUNG ALS MODULATOREN DES DOPAMIN D3 REZEPTORS**
PYRIDIN-2-ONE COMPOUNDS AND THEIR USE AS MODULATORS OF THE DOPAMINE D3 RECEPTOR
COMPOSES PYRIDIN-2-ONE ET LEUR UTILISATION EN TANT QUE MODULATEURS DU RECEPTEUR D3 DE LA DOPAMINE

(30) Priorität: 04.06.2004 DE 102004027359
(43) Veröffentlichungstag der Anmeldung: 21.03.2007
(73) Patentinhaber: Abbott GmbH & Co. KG, 65205 Wiesbaden (DE)
(72) Erfinder: GENESTE, Hervé, 67141 Neuhofen (DE); BRAJE, Wilfried, 68163 Mannheim (DE); HAUPT, Andreas, 68723 Schwetzingen (DE)
(74) Vertreter: Reitstötter - Kinzebach
(86) Internationale Anmeldenummer: PCT/EP2005/006001
(87) Internationale Veröffentlichungsnummer: WO 2005/118571

(56) Entgegenhaltungen:
- WO-A-03/002543
- WO-A-2004/080981
- US-A- 5 958 923
- US-A- 6 090 807
- US-B1- 6 342 604

## Beschreibung

Die vorliegende Erfindung betrifft neue Pyridin-2-onverbindungen der allgemeinen Formel I. Diese Verbindungen besitzen wertvolle therapeutische Eigenschaften und sind insbesondere zur Behandlung von Erkrankungen geeignet, die auf die Modulation des Dopamin-D₃-Rezeptors ansprechen.

Neuronen erhalten ihre Informationen unter anderem über G-Protein-gekoppelte Rezeptoren. Es gibt zahlreiche Substanzen, welche ihre Wirkung über diese Rezeptoren ausüben. Eine davon ist Dopamin. Es liegen gesicherte Erkenntnisse über die Anwesenheit von Dopamin und dessen physiologische Funktion als Neurotransmitter vor. Störungen im dopaminergen Transmittersystem resultieren in Erkrankungen des zentralen Nervensystems, zu denen z. B. Schizophrenie, Depression oder Parkinson-Krankheit zählen. Die Behandlung dieser und anderer Erkrankungen erfolgt mit Arzneimitteln, die mit den Dopaminrezeptoren in Wechselwirkung treten.

Bis 1990 waren zwei Subtypen von Dopaminrezeptoren pharmakologisch klar definiert, nämlich die D₁- und D₂-Rezeptoren. In jüngerer Zeit wurde ein dritter Subtyp gefunden, nämlich der D₃-Rezeptor, der einige Effekte der Antipsychotika und Anti-Parkinsonmittel zu vermitteln scheint (J.C. Schwartz et al., The Dopamine D3 Receptor as a Target for Antipsychotics, in Novel Antipsychotic Drugs, H.Y. Meltzer, Ed. Raven Press, New York 1992, Seiten 135-144; M. Dooley et al., Drugs and Aging 1998, 12, 495-514, J.N. Joyce, Pharmacology and Therapeutics 2001, 90, S. 231-59 "The Dopamine D3 Receptor as a Therapeutic Target for Antipsychotic and Antiparkinsonian Drugs").

Mittlerweile teilt man die Dopamin-Rezeptoren in zwei Familien ein. Einerseits die D₂-Gruppe bestehend aus D₂-, D₃- und D₄-Rezeptoren, andererseits die D₁-Gruppe bestehend aus D₁- und D₅-Rezeptoren. Während D₁- und D₂-Rezeptoren weit verbreitet sind, scheinen D₃-Rezeptoren hingegen regioselektiv exprimiert zu werden. So findet man diese Rezeptoren vorzugsweise im limbischen System, den Projektionsbereichen des mesolimbischen Dopaminsystems, vor allem im Nucleus accumbens, aber auch in anderen Bereichen, wie der Amygdala. Wegen dieser vergleichsweise regioselektiven Expression gelten D₃-Rezeptoren als nebenwirkungsarmes Target, und es wird angenommen, dass ein selektiver D₃-Ligand wohl die Eigenschaften bekannter Antipsychotika, nicht aber ihre Dopamin-D₂-Rezeptor-vermittelten neurologischen Nebenwirkungen haben sollte (P. Sokoloff et al., Localization and Function of the D3 Dopamine Receptor, Arzneim. Forsch./Drug Res. 42(1), 224 (1992); P. Sokoloff et al. Molecular Cloning and Characterization of a Novel Dopamine Receptor (D3) as a Target for Neuroleptics, Nature, 347, 146 (1990)).

Pyridinonverbindungen mit Dopamin-D₃-Rezeptoraffinität sind aus der WO 96/02246 bekannt. Diese Verbindungen weisen gute Affinitäten zum D₃-Rezeptor auf. Sie werden daher zur Behandlung von Erkrankungen des zentralen Nervensystems vorgeschlagen. Die Selektivität bezüglich anderer Rezeptoren ist jedoch nicht zufriedenstellend

Der Erfindung liegt daher die Aufgabe zu Grunde, Verbindungen zur Verfügung zu stellen, die als selektive Dopamin-D₃-Rezeptor-Liganden wirken. Diese Aufgabe wird gelöst durch Pyridin-2-onverbindungen der allgemeinen Formel I 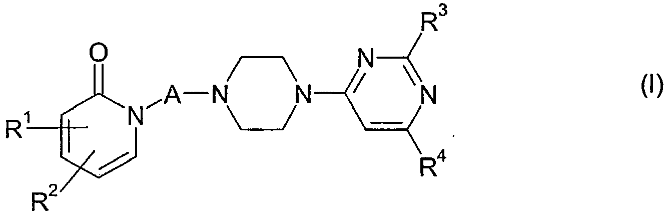 worin
- A: für eine 4- bis 6-gliedrige Kohlenwasserstoffkette steht, die 1 oder 2 Methylgruppen als Substituenten aufweisen kann, worin 1 oder 2 Kohlenstoffatome durch Sauerstoff, eine Carbonylgruppe oder Schwefel ersetzt sein können, und worin die Kohlenwasserstoffkette eine Doppelbindung oder eine Dreifachbindung aufweisen kann;
- R¹, R²: unabhängig voneinander für Wasserstoff, CN, NO₂, Halogen, OR⁵, NR⁶R⁷, C(O)NR⁶R⁷, O-C(O)NR⁶R⁷, SR⁸, SOR⁸, SO₂R⁸, SO₂NR⁶R⁷, COOR⁹, O-C(O)R¹⁰, COR¹⁰, C₁-C₆-Alkyl, C₁-C₆-Halogenalkyl, C₂-C₆-Alkenyl, C₂-C₆-Alkinyl, C₂-C₆-Halogenalkenyl, C₃-C₆-Cycloalkyl, 4- bis 6-gliedriges Heterocyclyl mit 1, 2 oder 3 Heteroatomen, ausgewählt unter O, S und N, das 1, 2 oder 3 Substituenten aufweisen kann, die unabhängig voneinander ausgewählt sind unter C₁-C₄-Alkyl, C₁-C₄-Alkoxy, NR⁶R⁷, CN, OH, C₁-C₂-Fluoralkyl oder Halogen, Phenyl, das 1, 2 oder 3 Substituenten aufweisen kann, die unabhängig voneinander ausgewählt sind unter C₁-C₄-Alkyl, C₁-C₄-Alkoxy, NR⁶R⁷, OH, CN, C₁-C₂-Fluoralkyl oder Halogen, C₁-C₆-Alkyl, das einen Substituenten aufweist, der ausgewählt ist unter OR⁵, NR⁶R⁷, C(O)NR⁶R⁷, O-C(O)NR⁶R⁷, SR⁸, SOR⁸, SO₂R⁸, SO₂NR⁶R⁷, COOR⁹, O-C(O)R¹⁰, COR¹⁰, C₃-C₆-Cycloalkyl, 5- oder 6-gliedrigem Heterocyclyl mit 1, 2 oder 3 Heteroatomen, ausgewählt unter O, S und N, und Phenyl, wobei Phenyl und Heterocyclyl 1, 2 oder 3 Substituenten aufweisen können, die unabhängig voneinander ausgewählt sind unter C₁-C₄-Alkyl, C₁-C₄-Alkoxy, NR⁶R⁷, CN, OH, C₁-C₂-Fluoralkyl oder Halogen, C₂-C₆-Alkenyl, das einen Substituenten, ausgewählt unter OR⁵, NR⁶R⁷, C(O)NR⁶R⁷, O-C(O)NR⁶R⁷, SR⁸, SOR⁸, SO₂R⁸, SO₂NR⁶R⁷, COOR⁹, O-C(O)R¹⁰, COR¹⁰, C₃-C₆-Cycloalkyl, 5- oder 6-gliedriges Heterocyclyl mit 1, 2 oder 3 Heteroatomen, ausgewählt unter O, S und N, und Phenyl aufweist, wobei Phenyl und Heterocyclyl ihrerseits 1, 2 oder 3 Substituenten aufweisen können, die unabhängig voneinander ausgewählt sind unter C₁-C₄-Alkyl, C₁-C₄-Alkoxy, NR⁶R⁷, OH, CN, C₁-C₂-Fluoralkyl oder Halogen, stehen;
- R³, R⁴: unabhängig voneinander für OR⁵, NR⁶R⁷, CN, C₁-C₆-Alkyl, das gegebenenfalls durch OH, C₁-C₄-Alkoxy, Halogen oder Phenyl ein- oder mehrfach substituiert ist, das seinerseits 1, 2 oder 3 Substituenten, ausgewählt unter C₁-C₄-Alkyl, C₁-C₄-Alkoxy, NR⁶R⁷, OH, CN, C₁-C₂-Fluoralkyl oder Halogen, tragen kann, C₂-C₆-Alkenyl, C₂-C₆-Alkinyl, C₃-C₆-Cycloalkyl, C₄-C₁₀-Bicycloalkyl, C₆-C₁₀-Tricycloalkyl, wobei die 5 letztgenannten Gruppen gegebenenfalls ein- oder mehrfach mit Halogen oder C₁-C₄-Alkyl substituiert sein können, Halogen, CN, C₁-C₄-Alkoxy, 5- oder 6-gliedriges Heterocyclyl mit 1, 2 oder 3 Heteroatomen, ausgewählt unter O, S und N, und Phenyl, wobei Phenyl und Heterocyclyl gegebenenfalls 1, 2 oder 3 Substituenten tragen, die unabhängig voneinander ausgewählt sind unter C₁-C₄-Alkyl, C₁-C₄-Alkoxy, NR⁶R⁷, CN, C₁-C₂-Fluoralkyl und Halogen, stehen;
- R⁵, R⁶, R⁷ R⁸, R⁹ und R¹⁰: unabhängig voneinander für H, C₁-C₆-Alkyl, das gegebenenfalls durch OH, C₁-C₄-Alkoxy oder Phenyl substituiert ist, das seinerseits 1, 2 oder 3 Substituenten, ausgewählt unter C₁-C₄-Alkyl, C₁-C₄-Alkoxy, NR⁶R⁷, OH, CN, C₁-C₂-Fluoralkyl oder Halogen, tragen kann, C₁-C₆-Halogenalkyl oder Phenyl, das seinerseits 1, 2 oder 3 Substituenten, ausgewählt unter C₁-C₄-Alkyl, C₁-C₄-Alkoxy, NR⁶R⁷, OH, CN, C₁-C₂-Fluoralkyl oder Halogen, tragen kann, stehen, wobei
- R⁷: auch eine Gruppe COR¹¹ bedeuten kann, und wobei
- R⁶ mit R⁷: auch gemeinsam mit dem Stickstoffatom, an das sie gebunden sind, einen 4-, 5- oder 6-gliedrigen, gesättigten oder ungesättigten Heterocyclus bilden können, der ein weiteres Heteroatom, ausgewählt unter O, S und NR¹² als Ringglied aufweisen kann, wobei R¹² für Wasserstoff oder C₁-C₄-Alkyl steht, und der durch 1, 2, 3 oder 4 Alkylgruppen substituiert sein kann; und
- R¹¹: für Wasserstoff, C₁-C₄-Alkyl oder Phenyl, das gegebenenfalls durch 1, 2 oder 3 Reste substituiert ist, die unabhängig voneinander ausgewählt sind unter C₁-C₄-Alkyl, C₁-C₄-Alkoxy, NR⁶R⁷, CN, C₁-C₂-Fluoralkyl oder Halogen, steht;

sowie die Tautomere der Verbindungen I, die physiologisch akzeptablen Salze der Verbindungen I und die physiologisch akzeptablen Salze der Tautomere der Verbindungen I.

Gegenstand der vorliegenden Erfindung sind daher die Verbindungen der allgemeinen Formel I, ihre Tautomere sowie die physiologisch verträglichen Salze der Verbindungen I und die physiologisch akzeptablen Salze der Tautomere von I.

Gegenstand der vorliegenden Erfindung ist auch die Verwendung von Verbindungen der allgemeinen Formel I und von den Tautomeren sowie die Verwendung der physiologisch akzeptablen Salze der Verbindungen I und der Tautomere zur Herstellung eines pharmazeutischen Mittels zur Behandlung von Erkrankungen, die auf die Beeinflussung durch Dopamin-D₃-Rezeptorantagonisten bzw. -agonisten ansprechen.

Zu den Erkrankungen, die auf die Beeinflussung durch Dopamin-D₃-Rezeptorantagonisten bzw. -agonisten ansprechen, zählen insbesondere Störungen und Erkrankungen des zentralen Nervensystems, insbesondere affektive Störungen, neurotische Störungen, Belastungs- und somatoformen Störungen und Psychosen, speziell Schizophrenie und Depression und weiterhin Nierenfunktionsstörungen, insbesondere Nierenfunktionsstörungen, die durch Diabetes mellitus hervorgerufen werden (siehe WO 00/67847).

Erfindungsgemäß verwendet man zur Behandlung der vorstehend genannten Indikationen wenigstens eine Verbindung der allgemeinen Formel I, ein Tautomer von I, ein physiologisch akzeptables Salz einer Verbindung I oder ein Salz eines Tautomers von I. Sofern die Verbindungen der Formel I ein oder mehrere Asymmetriezentren aufweisen, können auch Enantiomerengemische, insbesondere Racemate, Diastereomerengemische, Tautomerengemische, vorzugsweise jedoch die jeweiligen im Wesentlichen reinen Enantiomere, Diastereomere und Tautomere eingesetzt werden.

Als Tautomere können insbesondere Verbindungen der Formel I vorliegen, worin einer oder beide Reste R¹ oder R² für OH oder NHR⁶ stehen, worin R⁶ die zuvor genannten Bedeutungen aufweist.

Ebenfalls brauchbar sind physiologisch akzeptablen Salze der Verbindungen der Formel I und von den Tautomeren von I, vor allem Säureadditionssalze mit physiologisch verträglichen Säuren. Als physiologisch verträgliche organische und anorganische Säuren kommen beispielsweise Chlorwasserstoffsäure, Bromwasserstoffsäure, Phosphorsäure, Schwefelsäure, C₁-C₄-Alkylsulfonsäuren wie Methansulfonsäure, aromatische Sulfonsäuren wie Benzolsulfonsäure und Toluolsulfonsäure, Oxalsäure, Maleinsäure, Fumarsäure, Milchsäure, Weinsäure, Adipinsäure und Benzoesäure in Betracht. Weitere brauchbare Säuren sind in Fortschritte der Arzneimittelforschung, Band 10, Seiten 224 ff., Birkhäuser Verlag, Basel und Stuttgart, 1966, beschrieben.

Halogen steht hier und im Folgenden für Fluor, Chlor, Brom oder Iod.

Cₙ-Cₘ-Alkyl (auch in Resten wie Alkoxy, Alkoxyalkyl, Alkylthio, Alkylamino, Dialkylamino, Alkylcarbonyl etc.) bedeutet eine geradkettige oder verzweigte Alkylgruppe mit n bis m Kohlenstoffatomen, z.B. 1 bis 6 und insbesondere 1 bis 4 Kohlenstoffatomen. Beispiele für eine Alkylgruppe sind Methyl, Ethyl, n-Propyl, iso-Propyl, n-Butyl, 2-Butyl, iso-Butyl, tert-Butyl, n-Pentyl, 2-Pentyl, Neopentyl, n-Hexyl und dergleichen.

Die Alkylgruppe kann, sofern nichts Gegenteiliges angegeben ist, einen oder mehrere Substituenten aufweisen, die unabhängig voneinander ausgewählt sind unter OH, C₁-C₄-Alkoxy, Halogen oder Phenyl. Im Falle eines Halogensubstituenten kann die Alkylgruppe insbesondere 1, 2, 3 oder 4 Halogenatome umfassen, die sich an einem oder mehreren C-Atomen befinden können, vorzugsweise in α- oder ω-Position. Derartige Gruppen werden im Folgenden auch als Halogenalkyl bezeichnet. Bevorzugtes Halogenalkyl ist C₁-C₂-Fluoralkyl oder C₁-C₂-Fluorchloralkyl insbesondere CF₃, CHF₂, CF₂Cl, CH₂F, CH₂CF₃.

Im Falle von Hydroxy substituiertem Alkyl weist die Alkylgruppe insbesondere eine HydroxyGruppe auf wie z.B. Hydroxymethyl, 2-Hydroxyeth-1-yl, 2-Hydroxyprop-1-yl, 3-Hydroxy-prop-1-yl, 1-Hydroxyprop-2-yl, 2-Hydroxybut-1-yl, 3-Hydroxybut-1-yl, 4-Hydroxybut-1-yl, 1-Hydroxybut-2-yl, 1-Hydroxybut-3-yl, 2-Hydroxybut-3-yl, 1-Hydroxy-2-methylprop-3-yl, 2-Hydroxy-2-methylprop-3-yl oder 2-Hydroxymethylprop-2-yl, insbesondere 2-Hydroxyethyl.

Im Falle von Alkoxy-substituiertem Alkyl weist die Alkylgruppe insbesondere einen Alkoxysubstituenten auf. Diese Reste werden, abhängig von der Anzahl der Kohlenstoffatome auch als Cₙ-Cₘ-Alkoxy-Cₙ-Cₘ-alkyl bezeichnet und stehen z.B. für Methoxymethyl, Ethoxymethyl, 2-Methoxyethyl, 1-Methoxyethyl, 2-Ethoxyethyl, 1-Ethoxyethyl, n-Propoxymethyl, Isopropoxymethyl, n-Butoxymethyl, (1-Methylpropoxy)methyl, (2-Methylpropoxy)methyl, CH₂-OC(CH₃)₃, 2-(Methoxy)ethyl, 2-(Ethoxy)ethyl, 2-(n-Propoxy)ethyl, 2-(1-Methylethoxy)ethyl, 2-(n-Butoxy)ethyl, 2-(1-Methylpropoxy)ethyl, 2-(2-Methylpropoxy)ethyl, 2-(1,1-Dimethylethoxy)ethyl, 2-(Methoxy)propyl, 2-(Ethoxy)propyl, 2-(n-Propoxy)propyl, 2-(1-Methylethoxy)propyl, 2-(n-Butoxy)propyl, 2-(1-Methylpropoxy)propyl, 2-(2-Methylpropoxy)propyl, 2-(1,1-Dimethylethoxy)propyl, 3-(Methoxy)propyl, 3-(Ethoxy)propyl, 3-(n-Propoxy)propyl, 3-(1-Methylethoxy)propyl, 3-(n-Butoxy)propyl, 3-(1-Methylpropoxy)propyl, 3-(2-Methylpropoxy)propyl, 3-(1,1-Dimethylethoxy)propyl, 2-(Methoxy)butyl, 2-(Ethoxy)butyl, 2-(n-Propoxy)butyl, 2-(1-Methylethoxy)butyl, 2-(n-Butoxy)butyl, 2-(1-Methylpropoxy)butyl, 2-(2-Methylpropoxy)butyl, 2-(1,1-Dimethylethoxy)butyl, 3-(Methoxy)butyl, 3-(Ethoxy)butyl, 3-(n-Propoxy)butyl, 3-(1-Methylethoxy)butyl, 3-(n-Butoxy)butyl, 3-(1-Methylpropoxy)butyl, 3-(2-Methylpropoxy)butyl, 3-(1,1-Dimethylethoxy)butyl, 4-(Methoxy)butyl, 4-(Ethoxy)butyl, 4-(n-Propoxy)butyl, 4-(1-Methylethoxy)butyl, 4-(n-Butoxy)butyl, 4-(1-Methylpropoxy)butyl, 4-(2-Methylpropoxy)butyl oder 4-(1,1-Dimethylethoxy)butyl, vorzugsweise Methoxymethyl, Ethoxymethyl, 2-Methoxyethyl, 2-Ethoxyethyl, 2-(Methoxy)propyl, 2-(Ethoxy)propyl oder 3-(Methoxy)propyl, 3-(Ethoxy)propyl. Cycloalkyl steht insbesondere für C₃-C₆-Cycloalkyl, wie Cyclopropyl, Cyclobutyl, Cyclopentyl und Cyclohexyl. Bicycloalkyl steht für einen bicyclischen Kohlenwasserstoffrest mit 4 bis 10 C-Atomen wie Bicyclo[2.1.0]pentyl, Bicyclo[2.2.0]hexyl, Bicyclo[3.1.0]hexyl, Bicyclo[3.2.0]heptyl, Bicyclo[2.2.1]heptyl, Bicyclo[2.2.2]octyl, Bicyclo[3.2.1]octyl und dergleichen. Tricycloalkyl steht für einen tricycloaliphatischen Rest mit 6 bis 10 Kohlenstoffatomen, beispielsweise für Adamantyl.

Der Begriff "Alkylen" umfasst grundsätzlich geradkettige oder verzweigte Reste mit vorzugsweise mit 3 bis 10 und besonders bevorzugt 3 bis 8 Kohlenstoffatomen wie Prop-1,2-ylen, Prop-1,3-ylen, But-1,2-ylen, But-1,3-ylen, But-1,4-ylen, 2-Methylprop-1,3-ylen, Pent-1,2-ylen, Pent-1,3-ylen, Pent-1,4-ylen, Pent-1,5-ylen, Pent-2,3-ylen, Pent-2,4-ylen, 1-Methylbut-1,4-ylen, 2-Methylbut-1,4-ylen, Hex-1,3-ylen, Hex-2,4-ylen, Hex-1,4-ylen, Hex-1,5-ylen, Hex-1,6-ylen und dergleichen. C₀-Alkylen steht für eine Einfachbindung, C₁-Alkylen für Methylen und C₂-Alkylen für 1,1-Ethylen oder 1,2-Ethylen.

C₂-C₆-Alkenyl steht für einen einfach ungesättigten linearen oder verzweigten Kohlenwasserstoffrest mit 2, 3, 4, 5 oder 6 C-Atomen, z.B. für Vinyl, Allyl (2-Propen-1-yl), 1-Propen-1-yl, 2-Propen-2-yl, Methallyl (2-Methylprop-2-en-1-yl) und dergleichen. C₃-C₄-Alkenyl steht insbesondere für Allyl, 1-Methylprop-2-en-1-yl, 2-Buten-1-yl, 3-Buten-1-yl oder Methallyl.

C₂-C₆-Halogenalkenyl steht für eine Alkenylgruppe wie oben definiert, in der alle oder ein Teil, z.B. 1, 2, 3, 4 oder 5 der Wasserstoffatome durch Halogenatome, insbesondere durch Chlor oder Fluor ersetzt sind.

C₂-C₆-Alkinyl steht für einen Kohlenwasserstoffrest mit 2, 3, 4, 5 oder 6 C-Atomen, der eine Dreifachbindung aufweist, z.B. für Propargyl (2-Propin-1-yl), 1-Methylprop-2-in-1-yl, 2-Butin-1-yl, 3-Butin-1-yl, 2-Pentin-1-yl, 1-Pentin-3-yl etc.

5- oder 6-gliedriges Heterocyclyl umfasst sowohl aromatisches Heterocyclyl (Hetaryl bzw. Heteroaryl) als auch vollständig gesättigte oder teilweise ungesättigte heterocyclische Reste. Heterocyclyl weist 1, 2 oder 3 Heteroatome, ausgewählt unter O, S und N auf, z. B. 1, 2 oder 3 Stickstoffatome, 1 oder 2 Sauerstoffatome, oder 1 Sauerstoffatom und 1 oder 2 Stickstoffatome oder 1 Schwefelatom und 1 oder 2 Stickstoffatome.

Heterocyclyl kann unsubstituiert sein oder 1, 2 oder 3 Substituenten aufweisen, die in der Regel unter C₁-C₄-Alkyl, C₁-C₄-Alkoxy, OH, CN, NR⁶R⁷, C₁-C₂-Fluoralkyl und Halogen ausgewählt sind.

Beispiele für gesättigtes Heterocyclyl sind Pyrrolidinyl, Piperidinyl, Piperazinyl, Morpholinyl, Oxolanyl, 1,3-Dioxolanyl, 1,3- und 1,4-Dioxanyl, 1,3-Oxothiolanyl, Oxazolidinyl und dergleichen. Beispiele für "5- oder 6-gliedrige aromatische heterocyclische Reste", die 1, 2 oder 3 Heteroatome aufweisen, die ausgewählt sind unter O, S und N, sind vor allem Pyridinyl, Pyrimidinyl, Pyrazinyl, Triazinyl, Imidazolyl, Pyrrolyl, Pyrazolyl, Thienyl, Furanyl, Oxazolyl, Thiazolyl, Isoxazolyl, Tetrazolyl, Thiadiazolyl und Triazolyl. Diese können an den Stickstoffatomen sowie an den Kohlenstoffatomen 1, 2 oder 3 der vorgenannten Substituenten aufweisen. Sofern einer der Substituenten Hydroxy ist, können die Reste auch in einer tautomeren Form mit Carbonylgruppe vorliegen.

In der Gruppe A befinden sich die beiden Bindungsstellen vorzugsweise in 1,4-Position, 1,5-Position oder 1,6-Position. Somit ist in den Verbindungen I der 2-Pyridonrest vom Piperazin-Rest vorzugsweise durch eine Kette von 4, 5 oder 6 Atomen getrennt. 1 oder 2 Kohlenstoffatome in der Kette A können durch Sauerstoff, Schwefel oder eine Carbonylgruppe ersetzt sein. Sofern ein oder zwei Kohlenstoffatome durch Sauerstoff oder Schwefel ersetzt sind, befinden sich diese Heteroatome vorzugsweise nicht an den Enden der Gruppe A und sind insbesondere nicht zueinander benachbart. A kann auch eine Doppel- oder Dreifachbindung und/oder 1 oder 2 Methylgruppen aufweisen und ist vorzugsweise gesättigt. Beispiele für Reste A sind CH₂-CH₂-CH₂-CH₂, CH₂-CH=CH-CH₂, CH₂-C=C-CH₂, CH₂-CH(CH₃)-CH₂-CH₂, etc.

Im Hinblick auf die Verwendung der erfindungsgemäßen Verbindungen als Dopamin-D₃-Rezeptor-Liganden weisen die Variablen A, R¹, R², R³ und R⁴ unabhängig voneinander vorzugsweise die nachstehend angegebenen Bedeutungen auf:
- R¹: Halogen, OR⁵, NR⁶R⁷, C₁-C₄-Alkyl, das gegebenenfalls durch OH, C₁-C₄-Alkoxy oder Halogen substituiert ist, aromatisches, 5- oder 6-gliedriges Heterocyclyl mit 1, 2 oder 3 Heteroatomen, ausgewählt unter O, S und N, das 1, 2 oder 3 Substituenten aufweisen kann, die unabhängig voneinander ausgewählt sind unter C₁-C₄-Alkyl, C₁-C₄-Alkoxy, NR⁶R⁷, CN, OH, C₁-C₂-Fluoralkyl oder Halogen, und Phenyl, das 1, 2 oder 3 Substituenten aufweisen kann, die unabhängig voneinander ausgewählt sind unter C₁-C₄-Alkyl, C₁-C₄-Alkoxy, NR⁶R⁷, OH, CN, C₁-C₂-Fluoralkyl oder Halogen. Insbesondere ist R¹ ausgewählt unter gegebenenfalls substituiertem Phenyl, Halogen, OH, NR⁶R⁷, C₁-C₄-Alkoxy und C₁-C₄-Alkyl, das gegebenenfalls durch OH, C₁-C₄-Alkoxy oder Halogen substituiert ist, besonders bevorzugt unter Phenyl, OH, Halogen, C₁-C₂-Alkoxy, C₁-C₂-Alkyl, C₁-C₂-Fluoralkyl, und speziell unter Phenyl, OH, Methyl, Methoxy und Trifluormethyl;
- R²: Wasserstoff, Halogen, CN, OR⁵, NR⁶R⁷, SR⁸, und C₁-C₄-Alkyl, das gegebenenfalls durch OH, C₁-C₄-Alkoxy oder Halogen substituiert ist, und speziell Wasserstoff; Hierunter sind solche Verbindungen I bevorzugt, worin wenigstens einer der Reste R¹ oder R² von Wasserstoff verschieden ist. Insbesondere weisen die Verbindungen I einen von Wasserstoff verschiedenen Substituenten R¹ in der 3-, 4- oder 6-Position des Pyridonrings auf.
- R³: C₁-C₆-Alkyl, insbesondere verzweigtes Alkyl mit 3 bis 6 C-Atomen, oder C₃-C₆-Cycloalkyl, besonders bevorzugt tertiär gebundenes Alkyl mit 3 bis 6 C-Atomen und speziell tert.-Butyl.
- R⁴: C₁-C₆-Alkyl, C₃-C₆-Cycloalkyl, das gegebenenfalls 1 oder 2 Substituenten, ausgewählt unter Chlor und Methyl aufweist, und C₁-C₂-Fluoralkyl. In einer ersten besonders bevorzugten Ausführungsform steht R⁴ für C₁-C₂-Fluoralkyl oder C₂-C₆-Alkyl, speziell für Trifluormethyl oder C₃-C₄-Alkyl wie n-Propyl, n-Butyl, iso-Propyl oder tert.-Butyl. Ganz besonders bevorzugt steht R⁴ für n-Propyl oder Trifluormethyl. In einer anderen besonders bevorzugten Ausführungsform steht R⁴ für C₃-C₆-Cycloalkyl, das gegebenenfalls 1 oder 2 Substituenten, ausgewählt unter Chlor und Methyl, aufweist und insbesondere für Cyclopropyl, Cyclobutyl, Cyclopentyl oder 1-Methylcyclopropyl.
- A: eine viergliedrige Kohlenwasserstoffkette, die 1 oder 2 Methylgruppen als Substituenten und/oder eine Doppelbindung aufweisen kann, insbesondere Butan-1,4-diyl, 2-Methylbutan-1,4-diyl, (R)-2-Methylbutan-1,4-diyl, (S)-2-Methylbutan-1,4-diyl, 2-Methylbut-2-en-1,4-diyl, 3-Methylbut-2-en-1,4-diyl und 3-Methylbutan-1,4-diyl, (R)-3-Methylbutan-1,4-diyl, (S)-3-Methylbutan-1,4-diyl, besonders bevorzugt Butan-1,4-diyl.

Im Übrigen weisen die Gruppen R⁵, R⁶, R⁷, R⁸, R⁹, R¹⁰, R¹¹ und R¹² vorzugsweise die nachfolgend angegebenen Bedeutungen auf:
- R⁵: H, C₁-C₄-Alkyl, CF₃, CHF₂ oder Phenyl. Insbesondere bevorzugt steht OR⁵ für C₁-C₄-Alkoxy, speziell Methoxy oder Ethoxy, Trifluormethoxy oder Phenoxy.
- R⁶: Wasserstoff oder Alkyl.
- R⁷: Wasserstoff, C₁-C₄-Alkyl, Phenyl, Benzyl oder eine Gruppe C(O)R¹¹. In Substituenten CONR⁶R⁷ steht R⁶ vorzugsweise für H oder C₁-C₄-Alkyl und R⁷ vorzugsweise für H, C₁-C₄-Alkyl oder COR¹¹. Insbesondere bevorzugt steht CONR⁶R⁷ für CONH₂, CONHCH₃, CON(CH₃)₂ oder C(O)NHC(O)CH₃. In Substituenten NR⁶R⁷ steht R⁶ vorzugsweise für H, C₁-C₄-Alkyl oder phenylsubstituiertes C₁-C₄-Alkyl und R⁷ für H, C₁-C₄-Alkyl oder COR¹¹. Insbesondere bevorzugt steht NR⁶R⁷ für NH₂, NHCH₃, N(CH₃)₂, NH-Benzyl oder NHCOCH₃. In Substituenten SO₂NR⁶R⁷ steht R⁶ vorzugsweise für H oder C₁-C₄-Alkyl und R⁷ vorzugsweise für H, C₁-C₄-Alkyl oder COR¹¹. Insbesondere bevorzugt steht SO₂NR⁶R⁷ für Sulfamoyl. In den vorgenannten Gruppen können R⁶ und R⁷ auch gemeinsam mit dem Stickstoffatom, an das sie gebunden sind, einen gesättigten 5- oder 6-gliedrigen, vorzugsweise gesättigten Stickstoffheterocyclus bilden, der ein weiteres Heteroatom wie N, S oder O aufweisen kann und der durch 1, 2, 3 oder 4 Alkylgruppen substituiert sein kann. Beispiele für derartige Heterocyclen sind Piperidinyl, Morpholinyl, Pyrrolidinyl, 4-Methylpiperazinyl und 4-Methylpiperidinyl.
- R⁸: H, C₁-C₄-Alkyl, Phenyl oder Benzyl. In Substituenten SR⁸ steht R⁸ vorzugsweise für H, C₁-C₄-Alkyl, Phenyl oder Benzyl. In Substituenten SOR⁸ steht R⁸ vorzugsweise Phenyl oder C₁-C₄-Alkyl. In Substituenten SO₂R⁸ steht R⁸ vorzugsweise für H oder C₁-C₄-Alkyl. Insbesondere bevorzugt steht SO₂R⁸ für Methylsulfonyl;
- R⁹: H oder C₁-C₄-Alkyl. Insbesondere bevorzugt steht COOR⁹ für C₁-C₄-Alkoxycarbonyl, wie Methoxycarbonyl, Ethoxycarbonyl, n-Propoxycarbonyl, i-Propoxycarbonyl, n-Butoxycarbonyl oder t-Butoxycarbonyl;
- R¹⁰: H, C₁-C₄-Alkyl oder Phenyl. Insbesondere bevorzugt steht COR¹⁰ für Formyl, Acetyl, Propionyl oder Benzoyl;
- R¹¹: H, C₁-C₄-Alkyl oder Phenyl. Insbesondere bevorzugt steht COR¹¹ für Formel, Acetyl, Propionyl oder Benzoyl;
- R¹²: H oder C₁-C₄-Alkyl.

Insbesondere bevorzugt sind die Verbindungen der im Folgenden angegebenen Formel la 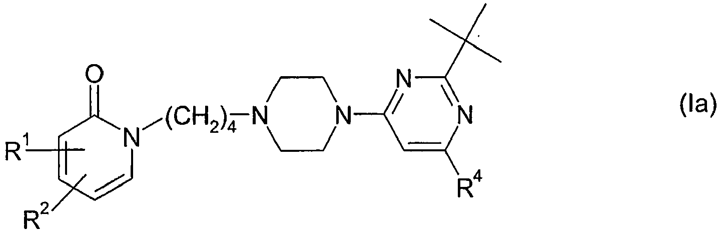 worin R¹, R² und R⁴ die zuvor und insbesondere die als bevorzugt genannten Bedeutungen haben, wobei R¹ vorzugsweise in der 3-, 4- oder 5-Postion des Pyridinonrings angeordnet ist. Beispiele für erfindungsgemäß bevorzugte Verbindungen I sind die Verbindungen der allgemeinen Formel Ia, worin R¹, R² und R⁴ die in jeweils einer Zeile der Tabelle 1 genannten Bedeutungen haben.

**Tabelle 1**

| | R¹, R² | R⁴ |
|---|---|---|
| 1. | 3-OH | CF₃ |
| 2. | 4-OH | CF₃ |
| 3. | 5-OH | CF₃ |
| 4. | 3-CH₃ | CF₃ |
| 5. | 4-CH₃ | CF₃ |
| 6. | 5-CH₃ | CF₃ |
| 7. | 3-OCH₃ | CF₃ |
| 8. | 4-OCH₃ | CF₃ |
| 9. | 5-OCH₃ | CF₃ |
| 10. | 3-N(CH₃)₂ | CF₃ |
| 11. | 4-N(CH₃)₂ | CF₃ |
| 12. | 5-N(CH₃)₂ | CF₃ |
| 13. | 3-Cl | CF₃ |
| 14. | 4-Cl | CF₃ |
| 15. | 5-Cl | CF₃ |
| 16. | 3-CF₃ | CF₃ |
| 17. | 4-CF₃ | CF₃ |
| 18. | 5-CF₃ | CF₃ |
| 19. | 3-CN | CF₃ |
| 20. | 4-CN | CF₃ |
| 21. | 5-CN | CF₃ |
| 22. | 3-H₃C-O-CH₂ | CF₃ |
| 23. | 4-H₃C-O-CH₂ | CF₃ |
| 24. | 5-H₃C-O-CH₂ | CF₃ |
| 25. | 6-CH₃ | CF₃ |
| 26. | 4-tert.-Butyl | CF₃ |
| 27. | 4-Azetidin-1-yl | CF₃ |
| 28. | 4-Pyrrolidin-1-yl | CF₃ |
| 29. | 4-Piperidin-1-yl | CF₃ |
| 30. | 4-Phenyl | CF₃ |
| 31. | 4-(1-Methylpyrrol-2-yl) | CF₃ |
| 32. | 4-(3-Pyridyl) | CF₃ |
| 33. | 4-(3-Thienyl) | CF₃ |
| 34. | 4-(4-Fluorphenyl) | CF₃ |
| 35. | 4-(4-Pyridyl) | CF₃ |
| 36. | 4-(3-Furyl) | CF₃ |
| 37. | 4-(2-Furyl) | CF₃ |
| 38. | 4-(2-Pyrrolyl) | CF₃ |
| 39. | 4-(2-Thienyl) | CF₃ |
| 40. | 4-(Pyridazin-2-yl) | CF₃ |
| 41. | 4-(4-Methylthiazol-5-yl) | CF₃ |
| 42. | 4-(2-Methyloxazol-4-yl) | CF₃ |
| 43. | 4-(Cyclopropyl) | CF₃ |
| 44. | 4-(Cyclobutyl) | CF₃ |
| 45. | 4-(Cyclopentyl) | CF₃ |
| 46. | 4-(Cyclohexyl) | CF₃ |
| 47. | 4-(Oxan-4-yl) | CF₃ |
| 48. | 4-(1-Methylpiperidin-4-yl) | CF₃ |
| 49. | 4-OH, 5-CF₃ | CF₃ |
| 50. | 4-OH, 5-CH₃ | CF₃ |
| 51. | 4-OH, 5-C₂H₅ | CF₃ |
| 52. | 4-OH, 5-CN | CF₃ |
| 53. | 4-OH, 5-F | CF₃ |
| 54. | 4-OH, 5-Cl | CF₃ |
| 55. | 4-OH, 6-CH₃ | CF₃ |
| 56. | 3-OH | CHF₂ |
| 57. | 4-OH | CHF₂ |
| 58. | 5-OH | CHF₂ |
| 59. | 3-CH₃ | CHF₂ |
| 60. | 4-CH₃ | CHF₂ |
| 61. | 5-CH₃ | CHF₂ |
| 62. | 3-OCH₃ | CHF₂ |
| 63. | 4-OCH₃ | CHF₂ |
| 64. | 5-OCH₃ | CHF₂ |
| 65. | 3-N(CH₃)₂ | CHF₂ |
| 66. | 4-N(CH₃)₂ | CHF₂ |
| 67. | 5-N(CH₃)₂ | CHF₂ |
| 68. | 3-Cl | CHF₂ |
| 69. | 4-Cl | CHF₂ |
| 70. | 5-Cl | CHF₂ |
| 71. | 3-CF₃ | CHF₂ |
| 72. | 4-CF₃ | CHF₂ |
| 73. | 5-CF₃ | CHF₂ |
| 74. | 3-CN | CHF₂ |
| 75. | 4-CN | CHF₂ |
| 76. | 5-CN | CHF₂ |
| 77. | 3-H₃C-O-CH₂ | CHF₂ |
| 78. | 4-H₃C-O-CH₂ | CHF₂ |
| 79. | 5-H₃C-O-CH₂ | CHF₂ |
| 80. | 6-CH₃ | CHF₂ |
| 81. | 4-tert.-Butyl | CHF₂ |
| 82. | 4-Azetidin-1-yl | CHF₂ |
| 83. | 4-Pyrrolidin-1-yl | CHF₂ |
| 84. | 4-Piperidin-1-yl | CHF₂ |
| 85. | 4-Phenyl | CHF₂ |
| 86. | 4-(1-Methylpyrrol-2-yl) | CHF₂ |
| 87. | 4-(3-Pyridyl) | CHF₂ |
| 88. | 4-(3-Thienyl) | CHF₂ |
| 89. | 4-(4-Fluorphenyl) | CHF₂ |
| 90. | 4-(4-Pyridyl) | CHF₂ |
| 91. | 4-(3-Furyl) | CHF₂ |
| 92. | 4-(2-Furyl) | CHF₂ |
| 93. | 4-(2-Pyrrolyl) | CHF₂ |
| 94. | 4-(2-Thienyl) | CHF₂ |
| 95. | 4-(Pyridazin-2-yl) | CHF₂ |
| 96. | 4-(4-Methylthiazol-5-yl) | CHF₂ |
| 97. | 4-(2-Methyloxazol-4-yl) | CHF₂ |
| 98. | 4-(Cyclopropyl) | CHF₂ |
| 99. | 4-(Cyclobutyl) | CHF₂ |
| 100. | 4-(Cyclopentyl) | CHF₂ |
| 101. | 4-(Cyclohexyl) | CHF₂ |
| 102. | 4-(Oxan-4-yl) | CHF₂ |
| 103. | 4-(1-Methylpiperidin-4-yl) | CHF₂ |
| 104. | 4-OH, 5-CF₃ | CHF₂ |
| 105. | 4-OH, 5-CH₃ | CHF₂ |
| 106. | 4-OH, 5-C₂H₅ | CHF₂ |
| 107. | 4-OH, 5-CN | CHF₂ |
| 108. | 4-OH, 5-F | CHF₂ |
| 109. | 4-OH, 5-Cl | CHF₂ |
| 110. | 4-OH, 6-CH₃ | CHF₂ |
| 111. | 3-OH | C(CH₃)₃ |
| 112. | 4-OH | C(CH₃)₃ |
| 113. | 5-OH | C(CH₃)₃ |
| 114. | 3-CH₃ | C(CH₃)₃ |
| 115. | 4-CH₃ | C(CH₃)₃ |
| 116. | 5-CH₃ | C(CH₃)₃ |
| 117. | 3-OCH₃ | C(CH₃)₃ |
| 118. | 4-OCH₃ | C(CH₃)₃ |
| 119. | 5-OCH₃ | C(CH₃)₃ |
| 120. | 3-N(CH₃)₂ | C(CH₃)₃ |
| 121. | 4-N(CH₃)₂ | C(CH₃)₃ |
| 122. | 5-N(CH₃)₂ | C(CH₃)₃ |
| 123. | 3-Cl | C(CH₃)₃ |
| 124. | 4-Cl | C(CH₃)₃ |
| 125. | 5-Cl | C(CH₃)₃ |
| 126. | 3-CF₃ | C(CH₃)₃ |
| 127. | 4-CF₃ | C(CH₃)₃ |
| 128. | 5-CF₃ | C(CH₃)₃ |
| 129. | 3-CN | C(CH₃)₃ |
| 130. | 4-CN | C(CH₃)₃ |
| 131. | 5-CN | C(CH₃)₃ |
| 132. | 3-H₃C-O-CH₂ | C(CH₃)₃ |
| 133. | 4-H₃C-O-CH₂ | C(CH₃)₃ |
| 134. | 5-H₃C-O-CH₂ | C(CH₃)₃ |
| 135. | 6-CH₃ | C(CH₃)₃ |
| 136. | 4-tert.-Butyl | C(CH₃)₃ |
| 137. | 4-Azetidin-1-yl | C(CH₃)₃ |
| 138. | 4-Pyrrolidin-1-yl | C(CH₃)₃ |
| 139. | 4-Piperidin-1-yl | C(CH₃)₃ |
| 140. | 4-Phenyl | C(CH₃)₃ |
| 141. | 4-(1-Methylpyrrol-2-yl) | C(CH₃)₃ |
| 142. | 4-(3-Pyridyl) | C(CH₃)₃ |
| 143. | 4-(3-Thienyl) | C(CH₃)₃ |
| 144. | 4-(4-Fluorphenyl) | C(CH₃)₃ |
| 145. | 4-(4-Pyridyl) | C(CH₃)₃ |
| 146. | 4-(3-Furyl) | C(CH₃)₃ |
| 147. | 4-(2-Furyl) | C(CH₃)₃ |
| 148. | 4-(2-Pyrrolyl) | C(CH₃)₃ |
| 149. | 4-(2-Thienyl) | C(CH₃)₃ |
| 150. | 4-(Pyridazin-2-yl) | C(CH₃)₃ |
| 151. | 4-(4-Methylthiazol-5-yl) | C(CH₃)₃ |
| 152. | 4-(2-Methyloxazol-4-yl) | C(CH₃)₃ |
| 153. | 4-(Cyclopropyl) | C(CH₃)₃ |
| 154. | 4-(Cyclobutyl) | C(CH₃)₃ |
| 155. | 4-(Cyclopentyl) | C(CH₃)₃ |
| 156. | 4-(Cyclohexyl) | C(CH₃)₃ |
| 157. | 4-(Oxan-4-yl) | C(CH₃)₃ |
| 158. | 4-(1-Methylpiperidin-4-yl) | C(CH₃)₃ |
| 159. | 4-OH, 5-CF₃ | C(CH₃)₃ |
| 160. | 4-OH, 5-CH₃ | C(CH₃)₃ |
| 161. | 4-OH, 5-C₂H₅ | C(CH₃)₃ |
| 162. | 4-OH, 5-CN | C(CH₃)₃ |
| 163. | 4-OH, 5-F | C(CH₃)₃ |
| 164. | 4-OH, 5-Cl | C(CH₃)₃ |
| 165. | 4-OH, 6-CH₃ | C(CH₃)₃ |
| 166. | 3-OH | *cyclo*-C₃H₅ |
| 167. | 4-OH | *cyclo*-C₃H₅ |
| 168. | 5-OH | *cyclo*-C₃H₅ |
| 169. | 3-CH₃ | *cyclo*-C₃H₅ |
| 170. | 4-CH₃ | *cyclo*-C₃H₅ |
| 171. | 5-CH₃ | *cyclo*-C₃H₅ |
| 172. | 3-OCH₃ | *cyclo*-C₃H₅ |
| 173. | 4-OCH₃ | *cyclo*-C₃H₅ |
| 174. | 5-OCH₃ | *cyclo*-C₃H₅ |
| 175. | 3-N(CH₃)₂ | *cyclo*-C₃H₅ |
| 176. | 4-N(CH₃)₂ | *cyclo*-C₃H₅ |
| 177. | 5-N(CH₃)₂ | *cyclo*-C₄H₇ |
| 178. | 3-Cl | *cyclo*-C₃H₅ |
| 179. | 4-Cl | *cyclo*-C₃H₅ |
| 180. | 5-Cl | *cyclo*-C₃H₅ |
| 181. | 3-CF₃ | *cyclo*-C₃H₅ |
| 182. | 4-CF₃ | *cyclo*-C₃H₅ |
| 183. | 5-CF₃ | *cyclo*-C₃H₅ |
| 184. | 3-CN | *cyclo*-C₃H₅ |
| 185. | 4-CN | *cyclo*-C₃H₅ |
| 186. | 5-CN | *cyclo*-C₃H₅ |
| 187. | 3-H₃C-O-CH₂ | *cyclo*-C₃H₅ |
| 188. | 4-H₃C-O-CH₂ | *cyclo*-C₃H₅ |
| 189. | 5-H₃C-O-CH₂ | *cyclo*-C₃H₅ |
| 190. | 6-CH₃ | *cyclo*-C₃H₅ |
| 191. | 4-tert.-Butyl | *cyclo*-C₃H₅ |
| 192. | 4-Azetidin-1-yl | *cyclo*-C₃H₅ |
| 193. | 4-Pyrrolidin-1-yl | *cyclo*-C₃H₅ |
| 194. | 4-Piperidin-1-yl | *cyclo*-C₃H₅ |
| 195. | 4-Phenyl | *cyclo*-C₃H₅ |
| 196. | 4-(1-Methylpyrrol-2-yl) | *cyclo*-C₃H₅ |
| 197. | 4-(3-Pyridyl) | *cyclo*-C₃H₅ |
| 198. | 4-(3-Thienyl) | *cyclo*-C₃H₅ |
| 199. | 4-(4-Fluorphenyl) | *cyclo*-C₃H₅ |
| 200. | 4-(4-Pyridyl) | *cyclo*-C₃H₅ |
| 201. | 4-(3-Furyl) | *cyclo*-C₃H₅ |
| 202. | 4-(2-Furyl) | *cyclo*-C₃H₅ |
| 203. | 4-(2-Pyrrolyl) | *cyclo*-C₃H₅ |
| 204. | 4-(2-Thienyl) | *cyclo*-C₃H₅ |
| 205. | 4-(Pyridazin-2-yl) | *cyclo*-C₃H₅ |
| 206. | 4-(4-Methylthiazol-5-yl) | *cyclo*-C₃H₅ |
| 207. | 4-(2-Methyloxazol-4-yl) | *cyclo*-C₃H₅ |
| 208. | 4-(Cyclopropyl) | *cyclo*-C₃H₅ |
| 209. | 4-(Cyclobutyl) | *cyclo*-C₃H₅ |
| 210. | 4-(Cyclopentyl) | *cyclo*-C₃H₅ |
| 211. | 4-(Cyclohexyl) | *cyclo*-C₃H₅ |
| 212. | 4-(Oxan-4-yl) | *cyclo*-C₃H₅ |
| 213. | 4-(1-Methylpiperidin-4-yl) | *cyclo*-C₃H₅ |
| 214. | 4-OH, 5-CF₃ | *cyclo*-C₃H₅ |
| 215. | 4-OH, 5-CH₃ | *cyclo*-C₃H₅ |
| 216. | 4-OH, 5-C₂H₅ | *cyclo*-C₃H₅ |
| 217. | 4-OH, 5-CN | *cyclo*-C₃H₅ |
| 218. | 4-OH, 5-F | *cyclo-C₃H₅* |
| 219. | 4-OH, 5-Cl | *cyclo*-C₃H₅ |
| 220. | 4-OH, 6-CH₃ | *cyclo*-C₃H₅ |
| 221. | 3-OH | *cyclo*-C₄H₇ |
| 222. | 4-OH | *cyclo*-C₄H₇ |
| 223. | 5-OH | *cyclo*-C₄H₇ |
| 224. | 3-CH₃ | *cyclo*-C₄H₇ |
| 225. | 4-CH₃ | *cyclo*-C₄H₇ |
| 226. | 5-CH₃ | *cyclo*-C₄H₇ |
| 227. | 3-OCH₃ | *cyclo*-C₄H₇ |
| 228. | 4-OCH₃ | *cyclo*-C₄H₇ |
| 229. | 5-OCH₃ | *cyclo*-C₄H₇ |
| 230. | 3-N(CH₃)₂ | *cyclo*-C₄H₇ |
| 231. | 4-N(CH₃)₂ | *cyclo*-C₄H₇ |
| 232. | 5-N(CH₃)₂ | *cyclo*-C₄H₇ |
| 233. | 3-CI | *cyclo*-C₄H₇ |
| 234. | 4-Cl | *cyclo*-C₄H₇ |
| 235. | 5-Cl | *cyclo*-C₄H₇ |
| 236. | 3-CF₃ | *cyclo*-C₄H₇ |
| 237. | 4-CF₃ | *cyclo*-C₄H₇ |
| 238. | 5-CF₃ | *cyclo*-C₄H₇ |
| 239. | 3-CN | *cyclo*-C₄H₇ |
| 240. | 4-CN | *cyclo*-C₄H₇ |
| 241. | 5-CN | *cyclo*-C₄H₇ |
| 242. | 3-H₃C-O-CH₂ | *cyclo*-C₄H₇ |
| 243. | 4-H₃C-O-CH₂ | *cyclo*-C₄H₇ |
| 244. | 5-H₃C-O-CH₂ | *cyclo*-C₄H₇ |
| 245. | 6-CH₃ | *cyclo*-C₄H₇ |
| 246. | 4-tert.-Butyl | *cyclo*-C₄H₇ |
| 247. | 4-Azetidin-1-yl | *cyclo*-C₄H₇ |
| 248. | 4-Pyrrolidin-1-yl | *cyclo*-C₄H₇ |
| 249. | 4-Piperidin-1-yl | *cyclo-C₄H₇* |
| 250. | 4-Phenyl | *cyclo*-C₄H₇ |
| 251. | 4-(1-Methylpyrrol-2-yl) | *cyclo*-C₄H₇ |
| 252. | 4-(3-Pyridyl) | *cyclo*-C₄H₇ |
| 253. | 4-(3-Thienyl) | *cyclo*-C₄H₇ |
| 254. | 4-(4-Fluorphenyl) | *cyclo*-C₄H₇ |
| 255. | 4-(4-Pyridyl) | *cyclo*-C₄H₇ |
| 256. | 4-(3-Furyl) | *cyclo*-C₄H₇ |
| 257. | 4-(2-Furyl) | *cyclo*-C₄H₇ |
| 258. | 4-(2-Pyrrolyl) | *cyclo*-C₄H₇ |
| 259. | 4-(2-Thienyl) | *cyclo*-C₄H₇ |
| 260. | 4-(Pyridazin-2-yl) | *cyclo*-C₄H₇ |
| 261. | 4-(4-Methylthiazol-5-yl) | *cyclo*-C₄H₇ |
| 262. | 4-(2-Methyloxazol-4-yl) | *cyclo*-C₄H₇ |
| 263. | 4-(Cyclopropyl) | *cyclo*-C₄H₇ |
| 264. | 4-(Cyclobutyl) | *cyclo*-C₄H₇ |
| 265. | 4-(Cyclopentyl) | *cyclo*-C₄H₇ |
| 266. | 4-(Cyclohexyl) | *cyclo*-C₄H₇ |
| 267. | 4-(Oxan-4-yl) | *cyclo*-C₄H₇ |
| 268. | 4-(1-Methylpiperidin-4-yl) | *cyclo*-C₄H₇ |
| 269. | 4-OH, 5-CF₃ | *cyclo*-C₄H₇ |
| 270. | 4-OH, 5-CH₃ | *cyclo-C₄H₇* |
| 271. | 4-OH, 5-C₂H₅ | *cyclo*-C₄H₇ |
| 272. | 4-OH, 5-CN | *cyclo*-C₄H₇ |
| 273. | 4-OH, 5-F | *cyclo*-C₄H₇ |
| 274. | 4-OH, 5-Cl | *cyclo*-C₄H₇ |
| 275. | 4-OH, 6-CH₃ | *cyclo*-C₄H₇ |
| 276. | 3-OH | *cyclo*-C₅H₉ |
| 277. | 4-OH | *cyclo*-C₅H₉ |
| 278. | 5-OH | *cyclo*-C₅H₉ |
| 279. | 3-CH₃ | *cyclo*-C₅H₉ |
| 280. | 4-CH₃ | *cyclo*-C₅H₉ |
| 281. | 5-CH₃ | *cyclo*-C₅H₉ |
| 282. | 3-OCH₃ | *cyclo*-C₅H₉ |
| 283. | 4-OCH₃ | *cyclo*-C₅H₉ |
| 284. | 5-OCH₃ | *cyclo*-C₅H₉ |
| 285. | 3-N(CH₃)₂ | *cyclo*-C₅H₉ |
| 286. | 4-N(CH₃)₂ | *cyclo*-C₅H₉ |
| 287. | 5-N(CH₃)₂ | *cyclo*-C₅H₉ |
| 288. | 3-CI | *cyclo*-C₅H₉ |
| 289. | 4-Cl | *cyclo*-C₅H₉ |
| 290. | 5-Cl | *cyclo*-C₅H₉ |
| 291. | 3-CF₃ | *cyclo*-C₅H₉ |
| 292. | 4-CF₃ | *cyclo*-C₅H₉ |
| 293. | 5-CF₃ | *cyclo*-C₅H₉ |
| 294. | 3-CN | *cyclo*-C₅H₉ |
| 295. | 4-CN | *cyclo*-C₅H₉ |
| 296. | 5-CN | *cyclo*-C₅H₉ |
| 297. | 3-H₃C-O-CH₂ | *cyclo*-C₅H₉ |
| 298. | 4-H₃C-O-CH₂ | *cyclo*-C₅H₉ |
| 299. | 5-H₃C-O-CH₂ | *cyclo*-C₅H₉ |
| 300. | 6-CH₃ | *cyclo*-C₅H₉ |
| 301. | 4-tert.-Butyl | *cyclo*-C₅H₉ |
| 302. | 4-Azetidin-1-yl | *cyclo*-C₅H₉ |
| 303. | 4-Pyrrolidin-1-yl | *cyclo*-C₅H₉ |
| 304. | 4-Piperidin-1-yl | *cyclo*-C₅H₉ |
| 305. | 4-Phenyl | *cyclo*-C₅H₉ |
| 306. | 4-(1-Methylpyrrol-2-yl) | *cyclo*-C₅H₉ |
| 307. | 4-(3-Pyridyl) | *cyclo*-C₅H₉ |
| 308. | 4-(3-Thienyl) | *cyclo*-C₅H₉ |
| 309. | 4-(4-Fluorphenyl) | *cyclo*-C₅H₉ |
| 310. | 4-(4-Pyridyl) | *cyclo*-C₅H₉ |
| 311. | 4-(3-Furyl) | *cyclo*-C₅H₉ |
| 312. | 4-(2-Fu ryl) | *cyclo*-C₅H₉ |
| 313. | 4-(2-pyrrolyl) | *cyclo*-C₅H₉ |
| 314. | 4-(2-Thienyl) | *cyclo*-C₅H₉ |
| 315. | 4-(Pyridazin-2-yl) | *cyclo*-C₅H₉ |
| 316. | 4-(4-Methylthiazol-5-yl) | *cyclo*-C₅H₉ |
| 317. | 4-(2-Methyloxazol-4-yl) | *cyclo*-C₅H₉ |
| 318. | 4-(Cyclopropyl) | *cyclo*-C₅H₉ |
| 319. | 4-(Cyclobutyl) | *cyclo*-C₅H₉ |
| 320. | 4-(Cyclopentyl) | *cyclo*-C₅H₉ |
| 321. | 4-(Cyclohexyl) | *cyclo*-C₅H₉ |
| 322. | 4-(Oxan-4-yl) | *cyclo*-C₅H₉ |
| 323. | 4-(1-Methylpiperidin-4-yl) | *cyclo*-C₅H₉ |
| 324. | 4-OH, 5-CF₃ | *cyclo*-C₅H₉ |
| 325. | 4-OH, 5-CH₃ | *cyclo*-C₅H₉ |
| 326. | 4-OH, 5-C₂H₅ | *cyclo*-C₅H₉ |
| 327. | 4-OH, 5-CN | *cyclo*-C₅H₉ |
| 328. | 4-OH, 5-F | *cyclo*-C₅H₉ |
| 329. | 4-OH, 5-Cl | *cyclo*-C₅H₉ |
| 330. | 4-OH, 6-CH₃ | *cyclo*-C₅H₉ |
| 331. | 3-OH | CH₃ |
| 332. | 4-OH | CH₃ |
| 333. | 5-OH | CH₃ |
| 334. | 3-CH₃ | CH₃ |
| 335. | 4-CH₃ | CH₃ |
| 336. | 5-CH₃ | CH₃ |
| 337. | 3-OCH₃ | CH₃ |
| 338. | 4-OCH₃ | CH₃ |
| 339. | 5-OCH₃ | CH₃ |
| 340. | 3-N(CH₃)₂ | CH₃ |
| 341. | 4-N(CH₃)₂ | CH₃ |
| 342. | 5-N(CH₃)₂ | CH₃ |
| 343. | 3-Cl | CH₃ |
| 344. | 4-Cl | CH₃ |
| 345. | 5-Cl | CH₃ |
| 346. | 3-CF₃ | CH₃ |
| 347. | 4-CF₃ | CH₃ |
| 348. | 5-CF₃ | CH₃ |
| 349. | 3-CN | CH₃ |
| 350. | 4-CN | CH₃ |
| 351. | 5-CN | CH₃ |
| 352. | 3-H₃C-O-CH₂ | CH₃ |
| 353. | 4-H₃C-O-CH₂ | CH₃ |
| 354. | 5-H₃C-O-CH₂ | CH₃ |
| 355. | 6-CH₃ | CH₃ |
| 356. | 4-tert.-Butyl | CH₃ |
| 357. | 4-Azetidin-1-yl | CH₃ |
| 358. | 4-Pyrrolidin-1-yl | CH₃ |
| 359. | 4-Piperidin-1-yl | CH₃ |
| 360. | 4-Phenyl | CH₃ |
| 361. | 4-(1-Methylpyrrol-2-yl) | CH₃ |
| 362. | 4-(3-Pyridyl) | CH₃ |
| 363. | 4-(3-Thienyl) | CH₃ |
| 364. | 4-(4-Fluorphenyl) | CH₃ |
| 365. | 4-(4-Pyridyl) | CH₃ |
| 366. | 4-(3-Furyl) | CH₃ |
| 367. | 4-(2-Furyl) | CH₃ |
| 368. | 4-(2-Pyrrolyl) | CH₃ |
| 369. | 4-(2-Thienyl) | CH₃ |
| 370. | 4-(Pyridazin-2-yl) | CH₃ |
| 371. | 4-(4-Methylthiazol-5-yl) | CH₃ |
| 372. | 4-(2-Methyloxazol-4-yl) | CH₃ |
| 373. | 4-(Cyclopropyl) | CH₃ |
| 374. | 4-(Cyclobutyl) | CH₃ |
| 375. | 4-(Cyclopentyl) | CH₃ |
| 376. | 4-(Cyclohexyl) | CH₃ |
| 377. | 4-(Oxan-4-yl) | CH₃ |
| 378. | 4-(1-Methylpiperidin-4-yl) | CH₃ |
| 379. | 4-OH, 5-CF₃ | CH₃ |
| 380. | 4-OH, 5-CH₃ | CH₃ |
| 381. | 4-OH, 5-C₂H₅ | CH₃ |
| 382. | 4-OH, 5-CN | CH₃ |
| 383. | 4-OH, 5-F | CH₃ |
| 384. | 4-OH, 5-Cl | CH₃ |
| 385. | 4-OH, 6-CH₃ | CH₃ |
| 386. | 3-OH | CH(CH₃)₂ |
| 387. | 4-OH | CH(CH₃)₂ |
| 388. | 5-OH | CH(CH₃)₂ |
| 389. | 3-CH₃ | CH(CH₃)₂ |
| 390. | 4-CH₃ | CH(CH₃)₂ |
| 391. | 5-CH₃ | CH(CH₃)₂ |
| 392. | 3-OCH₃ | CH(CH₃)₂ |
| 393. | 4-OCH₃ | CH(CH₃)₂ |
| 394. | 5-OCH₃ | CH(CH₃)₂ |
| 395. | 3-N(CH₃)₂ | CH(CH₃)₂ |
| 396. | 4-N(CH₃)₂ | CH(CH₃)₂ |
| 397. | 5-N(CH₃)₂ | CH(CH₃)₂ |
| 398. | 3-Cl | CH(CH₃)₂ |
| 399. | 4-Cl | CH(CH₃)₂ |
| 400. | 5-Cl | CH(CH₃)₂ |
| 401. | 3-CF₃ | CH(CH₃)₂ |
| 402. | 4-CF₃ | CH(CH₃)₂ |
| 403. | 5-CF₃ | CH(CH₃)₂ |
| 404. | 3-CN | CH(CH₃)₂ |
| 405. | 4-CN | CH(CH₃)₂ |
| 406. | 5-CN | CH(CH₃)₂ |
| 407. | 3-H₃C-O-CH₂ | CH(CH₃)₂ |
| 408. | 4-H₃C-O-CH₂ | CH(CH₃)₂ |
| 409. | 5-H₃C-O-CH₂ | CH(CH₃)₂ |
| 410. | 6-CH₃ | CH(CH₃)₂ |
| 411. | 4-tert.-Butyl | CH(CH₃)₂ |
| 412. | 4-Azetidin-1-yl | CH(CH₃)₂ |
| 413. | 4-Pyrrolidin-1-yl | CH(CH₃)₂ |
| 414. | 4-Piperidin-1-yl | CH(CH₃)₂ |
| 415. | 4-Phenyl | CH(CH₃)₂ |
| 416. | 4-(1-Methylpyrrol-2-yl) | CH(CH₃)₂ |
| 417. | 4-(3-Pyridyl₎ | CH(CH₃)₂ |
| 418. | 4-(3-Thienyl) | CH(CH₃)₂ |
| 419. | 4-(4-Fluorphenyl) | CH(CH₃)₂ |
| 420. | 4-(4-Pyridyl) | CH(CH₃)₂ |
| 421. | 4-(3-Furyl) | CH(CH₃)₂ |
| 422. | 4-(2-Furyl) | CH(CH₃)₂ |
| 423. | 4-(2-Pyrrolyl) | CH(CH₃)₂ |
| 424. | 4-(2-Thienyl) | CH(CH₃)₂ |
| 425. | 4-(Pyridazin-2-yl) | CH(CH₃)₂ |
| 426. | 4-(4-Methylthiazol-5-yl) | CH(CH₃)₂ |
| 427. | 4-(2-Methyloxazol-4-yl) | CH(CH₃)₂ |
| 428. | 4-(Cyclopropyl) | CH(CH₃)₂ |
| 429. | 4-(Cyclobutyl) | CH(CH₃)₂ |
| 430. | 4-(Cyclopentyl) | CH(CH₃)₂ |
| 431. | 4-(Cyclohexyl) | CH(CH₃)₂ |
| 432. | 4-(Oxan-4-yl) | CH(CH₃)₂ |
| 433. | 4-(1-Methylpiperidin-4-yl) | CH(CH₃)₂ |
| 434. | 4-OH, 5-CF₃ | CH(CH₃)₂ |
| 435. | 4-OH, 5-CH₃ | CH(CH₃)₂ |
| 436. | 4-OH, 5-C₂H₅ | CH(CH₃)₂ |
| 437. | 4-OH, 5-CN | CH(CH₃)₂ |
| 438. | 4-OH, 5-F | CH(CH₃)₂ |
| 439. | 4-OH, 5-Cl | CH(CH₃)₂ |
| 440. | 4-OH, 6-CH₃ | CH(CH₃)₂ |
| 441. | 3-OH | CH₂CH₂CH₃ |
| 442. | 4-OH | CH₂CH₂CH₃ |
| 443. | 5-OH | CH₂CH₂CH₃ |
| 444. | 3-CH₃ | CH₂CH₂CH₃ |
| 445. | 4-CH₃ | CH₂CH₂CH₃ |
| 446. | 5-CH₃ | CH₂CH₂CH₃ |
| 447. | 3-OCH₃ | CH₂CH₂CH₃ |
| 448. | 4-OCH₃ | CH₂CH₂CH₃ |
| 449. | 5-OCH₃ | CH₂CH₂CH₃ |
| 450. | 3-N(CH₃)₂ | CH₂CH₂CH₃ |
| 451. | 4-N(CH₃)₂ | CH₂CH₂CH₃ |
| 452. | 5-N(CH₃)₂ | CH₂CH₂CH₃ |
| 453. | 3-Cl | CH₂CH₂CH₃ |
| 454. | 4-Cl | CH₂CH₂CH₃ |
| 455. | 5-Cl | CH₂CH₂CH₃ |
| 456. | 3-CF₃ | CH₂CH₂CH₃ |
| 457. | 4-CF₃ | CH₂CH₂CH₃ |
| 458. | 5-CF₃ | CH₂CH₂CH₃ |
| 459. | 3-CN | CH₂CH₂CH₃ |
| 460. | 4-CN | CH₂CH₂CH₃ |
| 461. | 5-CN | CH₂CH₂CH₃ |
| 462. | 3-H₃C-O-CH₂ | CH₂CH₂CH₃ |
| 463. | 4-H₃C-O-CH₂ | CH₂CH₂CH₃ |
| 464. | 5-H₃C-O-CH₂ | CH₂CH₂CH₃ |
| 465. | 6-CH₃ | CH₂CH₂CH₃ |
| 466. | 4-tert.-Butyl | CH₂CH₂CH₃ |
| 467. | 4-Azetidin-1-yl | CH₂CH₂CH₃ |
| 468. | 4-Pyrrolidin-1-yl | CH₂CH₂CH₃ |
| 469. | 4-Piperidin-1-yl | CH₂CH₂CH₃ |
| 470. | 4-Phenyl | CH₂CH₂CH₃ |
| 471. | 4-(1-Methylpyrrol-2-yl) | CH₂CH₂CH₃ |
| 472. | 4-(3-Pyridyl) | CH₂CH₂CH₃ |
| 473. | 4-(3-Thienyl) | CH₂CH₂CH₃ |
| 474. | 4-(4-Fluorphenyl) | CH₂CH₂CH₃ |
| 475. | 4-(4-Pyridyl) | CH₂CH₂CH₃ |
| 476. | 4-(3-Furyl) | CH₂CH₂CH₃ |
| 477. | 4-(2-Furyl) | CH₂CH₂CH₃ |
| 478. | 4-(2-Pyrrolyl) | CH₂CH₂CH₃ |
| 479. | 4-(2-Thienyl) | CH₂CH₂CH₃ |
| 480. | 4-(Pyridazin-2-yl) | CH₂CH₂CH₃ |
| 481. | 4-(4-Methylthiazol-5-yl) | CH₂CH₂CH₃ |
| 482. | 4-(2-Methyloxazol-4-yl) | CH₂CH₂CH₃ |
| 483. | 4-(Cyclopropyl) | CH₂CH₂CH₃ |
| 484. | 4-(Cyclobutyl) | CH₂CH₂CH₃ |
| 485. | 4-(Cyclopentyl) | CH₂CH₂CH₃ |
| 486. | 4-(Cyclohexyl) | CH₂CH₂CH₃ |
| 487. | 4-(Oxan-4-yl) | CH₂CH₂CH₃ |
| 488. | 4-(1-Methylpiperidin-4-yl) | CH₂CH₂CH₃ |
| 489. | 4-OH, 5-CF₃ | CH₂CH₂CH₃ |
| 490. | 4-OH, 5-CH₃ | CH₂CH₂CH₃ |
| 491. | 4-OH, 5-C₂H₅ | CH₂CH₂CH₃ |
| 492. | 4-OH, 5-CN | CH₂CH₂CH₃ |
| 493. | 4-OH, 5-F | CH₂CH₂CH₃ |
| 494. | 4-OH, 5-Cl | CH₂CH₂CH₃ |
| 495. | 4-OH, 6-CH₃ | CH₂CH₂CH₃ |

Insbesondere bevorzugt sind außerdem die Verbindungen der im Folgenden angegebenen Formeln Ib, Ic, Id und le, 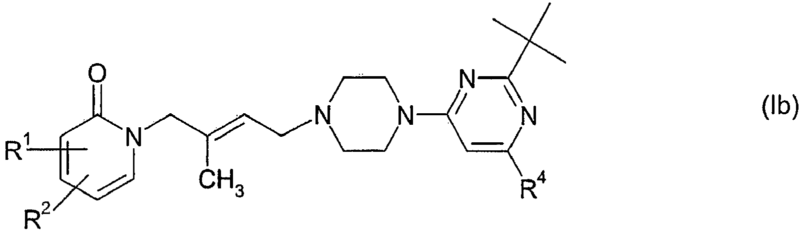 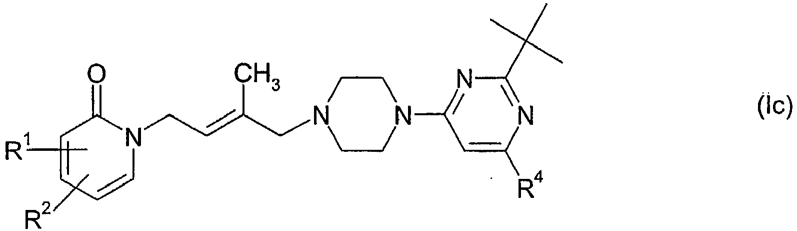 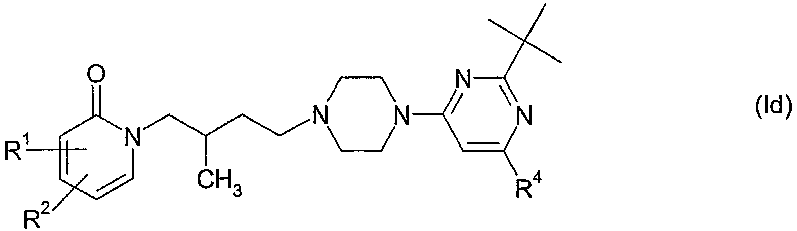 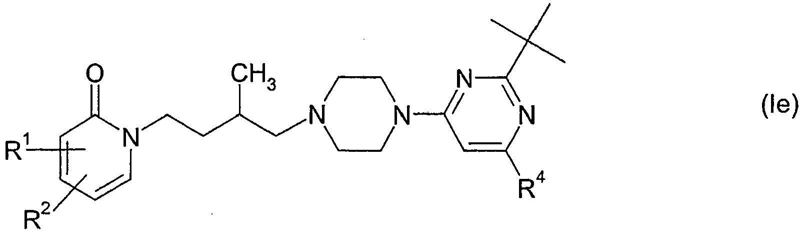 worin R¹, R² und R⁴ die zuvor und insbesondere die als bevorzugt genannten Bedeutungen haben, wobei R¹ vorzugsweise in der 3-, 4- oder 5-Postion des Pyridinonrings angeordnet ist. Beispiele für erfindungsgemäß bevorzugte Verbindungen I sind die Verbindungen der allgemeinen Formeln Ib, Ic, Id und Ie, worin R¹, R² und R⁴ die in jeweils einer Zeile der Tabelle 1 genannten Bedeutungen haben. In den Formeln Id und le kann das Kohlenstoffatom, welches die Methylgruppe trägt, sowohl S- als auch R-Konfiguration aufweisen. Die Formeln Id und le umfassen daher sowohl die Verbindungen mit einheitlicher S- oder R-Konfiguration als auch nicht-racemische Mischungen und Racemate.

Die Herstellung der erfindungsgemäßen Verbindungen I erfolgt analog zu literaturbekannten Methoden. Ein wichtiger Zugang zu den erfindungsgemäßen Verbindungen ist in Schema 1 dargestellt.

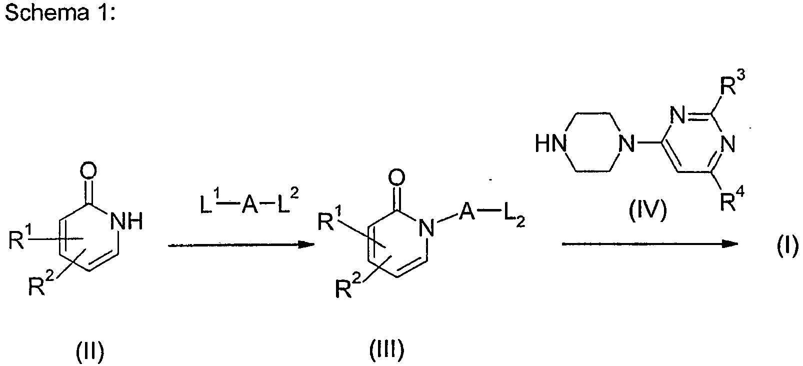

In Schema 1 haben R¹, R², R³, R und A die zuvor genannten Bedeutungen. L¹ und L² stehen für nucleophil verdrängbare Abgangsgruppen. Beispiele für geeignete nucleophil verdrängbare Abgangsgruppen sind Halogen, insbesondere Chlor, Brom oder Iod, Alkyl- und Arylsulfonat wie Mesylat, Tosylat. L¹ und L² sind vorzugsweise voneinander verschieden und weisen eine unterschiedliche Reaktivität auf. Beispielsweise steht L¹ für Brom oder Iod und L² für Chlor. Die für die Umsetzung erforderlichen Reaktionsbedingungen entsprechen den für nucleophile Substitutionen üblichen Reaktionsbedingungen.

Verbindungen der allgemeinen Formel IV sind entweder literaturbekannt, z.B. aus WO 96/02519, WO 97/25324, WO 99/02503 oder der in diesen Schriften zitierten Literatur oder können nach den dort beschriebenen Verfahren hergestellt werden.

Die Pyridinon-Verbindungen der Formeln II sind bekannt und teilweise kommerziell erhältlich oder können nach bekannten Verfahren zur Pyridinonsynthese hergestellt werden, wie z. B. beschrieben in J. Med. Chem. 16(5), 1973, S. 524-528, J. Org. Chem., 67, 2002, S. 4304-4308, Bioorg., Med. Chem. Lett, 12, 2002, S. 3537-3541.

In den Verbindungen I mit R¹ = SH kann die Thiolgruppe nach Standardverfahren der organischen Chemie in andere Reste R¹ umgewandelt werden. Eine Übersicht gibt das Schema 2.

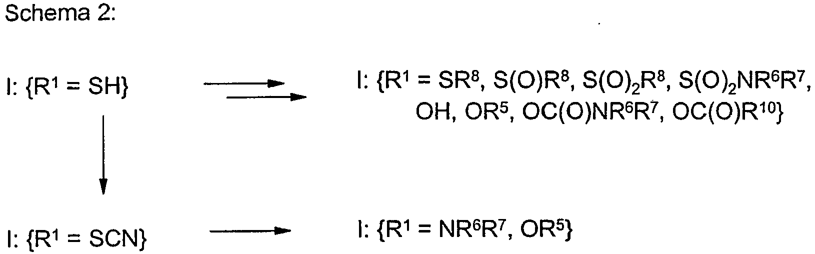

Verfahren hierzu sind dem Fachmann bekannt und umfassen die Umwandlung von SH in SR⁸ via Alkylierung, die Oxidation von SR⁸ zu den korrespondierenden Gruppen SOR⁸ und SO₂R⁶, den oxidativen Abbau von SH zu OH, mit gegebenenfalls sich anschließender Alkylierung oder Veresterung zu den Gruppen OR⁵, OC(O)NR⁶R⁷ oder OC(O)R¹⁰.

In den Verbindungen I sowie in den Ausgangsmaterialien der Formel II, worin R¹ für Cl, Br oder I steht, kann das Halogenatom in einer übergangsmetallkatalysierten Reaktion, z.B. in Gegenwart von elementarem Pd oder Pd-Verbindungen, z.B. in im Sinne einer Suzuki-Reaktion, einer Stille-Kupplung, oder einer Heck-Reaktion durch einen C-gebundenen organischen Rest R¹ substituiert werden. Insbesondere kann man Verbindungen I sowie Pyridone II, worin R¹ für einen gegebenenfalls substituierten Phenylring steht, durch Umsetzung der entsprechenden Halogenverbindung I bzw. II (R¹ = Cl, Br oder I) mit einem Borat M[Aryl₄B], worin M für ein Kation eines Alkalimetalls, z.B. für Na⁺ steht und Aryl gegebenenfalls substituiertes Phenyl bedeutet, unter Suzuki-Bedingungen (siehe Tetrahedron 1997, 53, 14437-50) herstellen. Diese modifizierte Suzuki-Kreuzkupplung zwischen einem Halogenpyridon I bzw. II und dem Borat erfolgt üblicherweise in wässrigen Lösungsmitteln in Gegenwart eines phosphinfreien Pd-Katalysators wie Palladium(II)-chlorid und in Gegenwart einer Base. Geeignete Basen sind beispielsweise Alkalimetallhydroxide wie Natriumhydroxid. Die Halogenpyridone II und Borate sind literaturbekannt.

Sofern nichts anderes angegeben wird, erfolgen die oben beschriebenen Umsetzungen im allgemeinen in einem Lösungsmittel bei Temperaturen zwischen Raumtemperatur und der Siedetemperatur des verwendeten Lösungsmittels. Brauchbare Lösungsmittel sind beispielsweise Ether, wie Diethylether, Diisopropylether, Methyl-tert.-butylether oder Tetrahydrofuran, Dimethylformamid, Dimethylsulfoxid, Dimethoxyethan, Toluol, Xylol, Acetonitril, Ketone, wie Aceton oder Methylethylketon, oder Alkohole, wie Methanol, Ethanol oder Butanol.

Die zur Umsetzung erforderliche Aktivierungsenergie kann mittels Mikrowellen in die Reaktionsmischung eingetragen werden (zur Umsetzung unter Einsatz von Mikrowellen, siehe Tetrahedron 2001, 57, S. 9199 ff, S. 9225 ff sowie allgemein "Microwaves in Organic Synthesis", André Loupy (Hrsg.), Wiley-VCH 2002).

Gewünschtenfalls arbeitet man in Gegenwart einer Base zur Neutralisation von bei den Umsetzungen freiwerdenden Protonen. Geeignete Basen umfassen anorganische Basen, wie Natrium- oder Kaliumcarbonat, Natrium- oder Kaliumhydrogencarbonat, weiterhin Alkoholate wie Natriummethylat, Natriumethylat, Alkalimetallhydride wie Natriumhydrid, metallorganische Verbindungen, wie Butyllithium- oder Alkylmagnesium-Verbindungen, oder organische Stickstoffbasen, wie Triethylamin oder Pyridin. Letztere können gleichzeitig als Lösungsmittel dienen.

Die Isolierung des Rohprodukts erfolgt in üblicher Weise, beispielsweise durch Filtration, Abdestillieren des Lösungsmittels oder Extraktion aus dem Reaktionsgemisch etc. Die Reinigung der erhaltenen Verbindungen kann in üblicher Weise erfolgen, beispielsweise durch Umkristallisieren aus einem Lösungsmittel, Chromatographie oder durch Überführen in eine Säureadditionssalz.

Die Säureadditionssalze werden in üblicher Weise durch Mischen der freien Base mit der entsprechenden Säure, gegebenenfalls in Lösung in einem organischen Lösungsmittel, beispielsweise einem niedermolekularen Alkohol, wie Methanol, Ethanol oder Propanol, einem Ether, wie Methyl-tert-butylether, Diisopropylether, einem Keton, wie Aceton oder Methylethylketon oder einem Ester, wie Essigsäureethylester, hergestellt.

Bei den erfindungsgemäßen Verbindungen der Formel I handelt es sich in der Regel um hochselektive Dopamin-D₃-Rezeptor-Liganden, die auf Grund ihrer geringen Affinität gegenüber anderen Rezeptoren wie D₁-Rezeptoren, D₄-Rezeptoren, α1- und/oder α2-adrenergen-Rezeptoren, muskarinergen Rezeptoren, histaminischen Rezeptoren, Opiat-Rezeptoren und insbesondere gegenüber Dopamin-D₂-Rezeptoren, nebenwirkungsärmer als die klassischen Neuroleptika sind, bei denen es sich um D₂-Rezeptor-Antagonisten handelt.

Die hohe Affinität der erfindungsgemäßen Verbindungen gegenüber D₃-Rezeptoren spiegelt sich in sehr geringen in vitro Kᵢ-Werte von in der Regel weniger als 100 nM (nmol/l), häufig weniger als 50 nM und vor allem von weniger als 10 nM wieder. Beispielsweise können Bindungsaffinitäten zu D₃-Rezeptoren in Rezeptorbindungsstudien über die Verdrängung von [¹²⁵I]-Iodosulpirid bestimmt werden.

Erfindungsgemäß von besonderer Bedeutung sind Verbindungen deren Selektivität Kᵢ(D₂)/Kᵢ(D₃) in der Regel wenigstens 10, häufig wenigstens 30, vorteilhaft wenigstens 50 beträgt. Rezeptorbindungsstudien an D₁-, D₂- und D₄-Rezeptoren können beispielsweise über die Verdrängung von [³H]SCH23390, [¹²⁵I]Iodosulpirid bzw. [¹²⁵I]Spiperon vorgenommen werden.

Die Verbindungen sind aufgrund ihres Bindungsprofils zur Behandlung von Erkrankungen brauchbar, die auf Dopamin-D₃-Liganden ansprechen, d. h. sie sind zur Behandlung von solchen Störungen bzw. Erkrankungen wirksam, bei denen eine Beeinflussung (Modulation) der Dopamin-D₃-Rezeptoren zur Verbesserung des Krankheitsbilds oder zum Ausheilen der Krankheit führt. Derartige Erkrankungen sind z. B. Störungen oder Erkrankungen des zentralen Nervensystems.

Unter Störungen bzw. Erkrankungen des zentralen Nervensystems versteht man Störungen, die das Rückenmark und vor allem das Gehirn betreffen. Der Begriff "Störung" im erfindungsgemäßen Sinne bezeichnet Anomalien, die in der Regel als krankhafte Zustände bzw. Funktionen angesehen werden, und sich in Form bestimmter Anzeichen, Symptome und/oder Fehlfunktionen zu erkennen geben können. Die erfindungsgemäße Behandlung kann auf einzelne Störungen, d. h. Anomalien bzw. krankhafte Zustände gerichtet sein, es können aber auch mehrere, gegebenenfalls ursächlich miteinander verbundene Anomalien zu Mustern, d.h. Syndromen, zusammengefasst sein, die erfindungsgemäß behandelt werden können.

Zu den erfindungsgemäß behandelbaren Störungen gehören vor allem psychiatrische und neurologische Störungen. Hierzu zählen insbesondere organische Störungen, symptomatische Störungen eingeschlossen, wie Psychosen vom akuten exogenen Reaktionstyp oder Begleit-Psychosen organischer bzw. exogener Ursache, z. B. bei Stoffwechselstörungen, Infektionen und Endokrinopathien; endogene Psychosen, wie Schizophrenie sowie schizotype und wahnhafte Störungen; affektive Störungen, wie Depressionen, Manie bzw. manisch-depressive Zustände; sowie Mischformen der zuvor geschilderten Störungen; neurotische und somatoforme Störungen sowie Störungen bei Belastung; dissoziative Störungen, z.B. Bewusstseinsstörungen, -ausfälle, -eintrübungen und -spaltungen und Persönlichkeitsstörungen; Störungen von Aufmerksamkeit und Wach/Schlafverhalten, wie Verhaltensstörungen und emotionale Störungen, deren Beginn in der Kindheit und Jugend liegt, z.B. Hyperaktivität bei Kindern, intellektuelle Defizite, insbesondere Aufmerksamkeitsstörungen (attention deficit disorders), Gedächtnis- und kognitive Störungen, z.B. Lern- und Gedächtnisschwäche (impaired cognitive function), Demenz, Narkolepsie und Schlafstörungen, z.B. restless legs syndrome; Entwicklungsstörungen; Angstzustände; Delirium; Störungen des Sexuallebens, z.B. Impotenz des Mannes; Essstörungen, z.B. Anorexie oder Bulimie; Sucht; und weitere nicht näher bezeichnete psychiatrische Störungen.

Zu den erfindungsgemäß behandelbaren Störungen gehören auch Parkinson und Epilepsie und insbesondere die damit in Zusammenhang stehenden affektiven Störungen.

Zu Suchterkrankungen gehören die durch den Missbrauch von psychotropen Substanzen, wie Arzneimittel oder Drogen, verursachten psychischen Störungen und Verhaltensstörungen sowie andere Suchterkrankungen, wie beispielsweise die Spielsucht und die Impulsive-Kontrollstörung (Impulse Control Disorders not elsewhere classified). Suchterzeugende Substanzen sind beispielsweise: Opioide (z. B. Morphin, Heroin, Codein); Kokain; Nikotin; Alkohol; Substanzen, die mit dem GABA-Chlorid-Kanal-Komplex interagieren, Sedativa, Hypnotika oder Tranquilizer, beispielsweise Benzodiazepine; LSD; Cannabinoide; psychomotorische Stimulanzien, wie 3,4-Methylendioxy-N-methylamphetamin (Ecstasy); Amphetamin und amphetaminartigen Substanzen wie Methylphenidat oder sonstige Stimulanzien einschließlich Koffein. Als suchterzeugende Substanzen kommen insbesondere Opioide, Kokain, Amphetamin oder amphetaminartige Substanzen, Nikotin und Alkohol in Betracht.

Im Hinblick auf die Behandlung von Suchterkrankungen sind unter den erfindungsgemäßen Verbindungen der Formel I solche besonders bevorzugt, die selbst keine psychotrope Wirkung besitzen. Dies kann im Test auch an Ratten beobachtet werden, die nach Verabreichung erfindungsgemäß brauchbarer Verbindungen die Selbstverabreichung von psychotropen Substanzen, beispielsweise Kokain, drosseln.

Gemäß einem weiteren Aspekt der vorliegenden Erfindung sind die erfindungsgemäßen Verbindungen zur Behandlung von Störungen geeignet, deren Ursachen zumindest zum Teil auf eine anomale Aktivität von Dopamin-D₃-Rezeptoren zurückzuführen sind.

Gemäß einem anderen Aspekt der vorliegenden Erfindung richtet sich die Behandlung vor allem auf diejenigen Störungen, die sich über eine Bindung von vorzugsweise exogen zugesetzten Bindungspartnern (Liganden) an Dopamin-D₃-Rezeptoren im Sinne einer zweckmäßigen medizinischen Behandlung beeinflussen lassen.

Die mit den erfindungsgemäßen Verbindungen behandelbaren Erkrankungen sind häufig gekennzeichnet durch eine progressive Entwicklung, d.h. die vorstehend beschriebenen Zustände verändern sich im Laufe der Zeit, in der Regel nimmt der Schweregrad zu und gegebenenfalls können Zustände ineinander übergehen oder weitere Zustände zu bereits bestehenden Zuständen hinzutreten.

Durch die erfindungsgemäßen Verbindungen lassen sich eine Vielzahl von Anzeichen, Symptomen und/oder Fehlfunktionen behandeln, die mit den Störungen des zentralen Nervensystems und insbesondere den vorstehend genannten Zuständen zusammenhängen. Hierzu gehören beispielsweise ein gestörter Realitätsbezug, mangelnde Einsicht und Fähigkeit, üblichen sozialen Normen bzw. Lebensanforderungen zu genügen, Wesensveränderungen, Veränderungen der Einzeltriebe, wie Hunger, Schlaf, Durst, etc., und der Stimmungslage, Störungen der Merk- und Kombinationsfähigkeit, Persönlichkeitsveränderungen, insbesondere Affektlabilität, Halluzinationen, Ich-Störungen, Zerfahrenheit, Ambivalenz, Autismus, Depersonalisation bzw. Sinnestäuschungen, Wahnideen, skandierende Sprache, fehlende Mitbewegung, kleinschrittiger Gang, Beugehaltung von Rumpf und Gliedern, Tremor, Mimikarmut, monotone Sprache, Depressionen, Apathie, erschwerte Spontanität und Entschlusskraft, verarmte Assoziationsfähigkeit, Angst, nervöse Unruhe, Stottern, soziale Phobie, Panikstörungen, Entzugssyndrome bei Abhängigkeit, maniforme Syndrome, Erregungs- und Verwirrtheitszustände, Dysphorie, dyskinetische Syndrome und Tic-Störungen, z.B. Chorea-Huntington, Gilles-de-la-Tourette-Syndrom, Schwindelsyndrome, z.B. peripherer Lage-, Dreh- und Schwankschwindel, Melancholie, Hysterie, Hypochondrie und ähnliches.

Eine Behandlung im erfindungsgemäßen Sinne umfasst nicht nur die Behandlung akuter oder chronischer Anzeichen, Symptome und/oder Fehlfunktionen sondern auch eine vorbeugende Behandlung (Prophylaxe), insbesondere als Rezidiv- oder Phasen-Prophylaxe. Die Behandlung kann symptomatisch, beispielsweise als Symptomsuppression ausgerichtet sein. Sie kann kurzzeitig erfolgen, mittelfristig ausgerichtet sein, oder es kann sich auch um eine Langzeitbehandlung, beispielsweise im Rahmen einer Erhaltungstherapie, handeln.

Vorzugsweise eignen sich die erfindungsgemäßen Verbindungen zur Behandlung von Erkrankungen des zentralen Nervensystems, insbesondere zur Behandlung von affektiven Störungen; neurotischen, Belastungs- und somatoformen Störungen und Psychosen und speziell zur Behandlung der Schizophrenie und der Depression. Aufgrund ihrer hohen Selektivität bezüglich des D₃-Rezeptors sind die erfindungsgemäßen Verbindungen I auch zur Behandlung von Nierenfunktionsstörungen, insbesondere von Nierenfunktionsstörungen, die durch Diabetes mellitus hervorgerufen wird (siehe WO 00/67847).

Die erfindungsgemäße Verwendung der beschriebenen Verbindungen beinhaltet im Rahmen der Behandlung ein Verfahren. Dabei wird dem zu behandelnden Individuum, vorzugsweise einem Säuger, insbesondere einem Menschen, Nutz- oder Haustier, eine wirksame Menge eines oder mehrerer Verbindungen, in der Regel der pharmazeutischen und tierarzneilichen Praxis entsprechend formuliert, verabreicht. Ob eine solche Behandlung angezeigt ist und in welcher Form sie zu erfolgen hat, hängt vom Einzelfall ab und unterliegt einer medizinischen Beurteilung (Diagnose), die vorhandene Anzeichen, Symptome und/oder Fehlfunktionen, Risiken, bestimmte Anzeichen, Symptome und/oder Fehlfunktionen zu entwickeln, und weitere Faktoren miteinbezieht.

Die Behandlung erfolgt in der Regel durch einmalige oder mehrmalige tägliche Verabreichung, gegebenenfalls zusammen oder im Wechsel mit anderen Wirkstoffen oder wirkstoffhaltigen Präparaten, so dass einem zu behandelnden Individuum eine Tagesdosis von vorzugsweise etwa 0,1 bis 1000 mg/kg Körpergewicht bei oraler Gabe bzw. von etwa 0,1 bis 100 mg/kg Körpergewicht bei parenteraler Gabe zugeführt wird.

Die Erfindung betrifft auch die Herstellung pharmazeutischer Mittel zur Behandlung eines Individuums, vorzugsweise eines Säugers, insbesondere eines Menschen, Nutz- oder Haustieres. So werden die Liganden gewöhnlich in Form von pharmazeutischen Zusammensetzungen verabreicht, die einen pharmazeutisch verträglichen Exzipienten mit wenigstens einem erfindungsgemäßen Liganden und gegebenenfalls weiteren Wirkstoffen umfassen. Diese Zusammensetzungen können beispielsweise auf oralem, rektalem, transdermalem, subkutanem, intravenösem, intramuskulärem oder intranasalem Weg verabreicht werden.

Beispiele geeigneter pharmazeutischer Formulierungen sind feste Arzneiformen, wie Pulver, Puder, Granulate, Tabletten, insbesondere Filmtabletten, Pastillen, Sachets, Cachets, Dragees, Kapseln wie Hart- und Weichgelatinekapseln, Suppositorien oder vaginale Arzneiformen, halbfeste Arzneiformen, wie Salben, Cremes, Hydrogele, Pasten oder Pflaster, sowie flüssige Arzneiformen, wie Lösungen, Emulsionen, insbesondere Öl-in-Wasser-Emulsionen, Suspensionen, beispielsweise Lotionen, Injektions- und Infusionszubereitungen, Augen- und Ohrentropfen. Auch implantierte Abgabevorrichtungen können zur Verabreichung erfindungsgemäßer Verbindungen verwendet werden. Ferner können auch Liposomen oder Mikrosphären zur Anwendung kommen.

Bei der Herstellung der Zusammensetzungen werden erfindungsgemäße Verbindungen gewöhnlich mit einem Exzipienten vermischt oder verdünnt. Exzipienten können feste, halbfeste oder flüssige Materialien sein, die als Vehikel, Träger oder Medium für den Wirkstoff dienen.

Geeignete Exzipienten sind in den einschlägigen Arzneimonographien gelistet. Ferner können die Formulierungen pharmazeutisch akzeptable Träger oder übliche Hilfsstoffe, wie Gleitmittel; Netzmittel; emulgierende und suspendierende Mittel; konservierende Mittel; Antioxidantien; Antireizstoffe; Chelatbildner; Dragierhilfsmittel; Emulsionsstabilisatoren; Filmbildner; Gelbildner; Geruchsmaskierungsmittel; Geschmackskorrigentien; Harze; Hydrokolloide; Lösemittel; Lösungsvermittler; Neutralisierungsmittel; Permeationsbeschleuniger; Pigmente; quaternäre Ammoniumverbindungen; Rückfettungs- und Überfettungsmittel; Salben-, Creme- oder Öl-Grundstoffe; Silikon-Derivate; Spreithilfsmittel; Stabilisatoren; Sterilanzien; Suppositoriengrundlagen; Tabletten-Hilfsstoffe, wie Bindemittel, Füllstoffe, Gleitmittel, Sprengmittel oder Überzüge; Treibmittel; Trocknungsmittel; Trübungsmittel; Verdickungsmittel; Wachse; Weichmacher; Weißöle umfassen. Eine diesbezügliche Ausgestaltung beruht auf fachmännischem Wissen, wie beispielsweise in Fiedler, H.P., Lexikon der Hilfsstoffe für Pharmazie, Kosmetik und angrenzende Gebiete, 4. Auflage, Aulendorf: ECV-Editio-Kantor-Verlag, 1996, dargestellt ist.

Die nachfolgenden Beispiele dienen zur Erläuterung der Erfindung ohne sie zu begrenzen.

Die magnetischen Kernresonanzspektraleigenschaften (NMR) beziehen sich auf chemische Verschiebungen (δ), ausgedrückt in Teile pro Million (ppm). Die relative Fläche für die Verschiebungen in dem ¹H-NMR-Spektrum entspricht der Anzahl der Wasserstoffatome für einen bestimmten funktionalen Typ in dem Molekül. Die Art der Verschiebung hinsichtlich der Multiplizität ist angegeben als Singulett (s), breites Singulett (s. br.), Dublett (d), breites Dublett (d br.), Triplett (t), breites Triplett (t br.), Quartett (q), Quintett (quint.), Multiplett (m).

### I. Herstellungsbeispiele:

### Beispiel 1: 1-(4-{4-[2-tert-Butyl-6-(trifluormethyl)pyrimidin-4-yl]piperazin-1-yl}butyl)-4-methylpyridin-2(1H)-on

### 1.1 1-(4-Chlorbutyl)-4-methylpyridin-2(1H)-on

Man rührte ein Gemisch aus 2-Hydroxy-4-methylpyridin (1,50 g, 13,75 mmol) und Kaliumcarbonat (1,90 g, 13,75 mmol) in 13 ml Methanol 15 Minuten bei Raumtemperatur und gab danach 1-Brom-4-chlorbutan (3,54 g, 20,62 mmol) und eine Spatelspitze Kalimiodid zu. Das Reaktionsgemisch erhitzte man 6 Stunden am Rückfluss und rührte danach 12 Stunden bei Raumtemperatur nach. Anschließend versetzte man das Reaktionsgemisch mit Wasser und extrahierte das wässrige Gemisch mit Dichlormethan. Nach dem Trocknen der organischen Phase und Abfiltrieren des Trockenmittels engte man die organische Phase im Vakuum ein. Die Flash-Chromatographie des erhaltenen Rückstands an Kieselgel (Eluierungsmittel: CH₂Cl₂/CH₃OH: 98:2) ergab 2,0 g 1-(4-Chlorbutyl)-4-methylpyridin-2(1*H*)-on.

¹H-NMR (400 MHz, CDCl₃) δ (ppm): 7,13 (1H, d), 6,37 (1H, s), 6,02 (1H, d), 3,95 (2H, t), 3,56 (2H, t), 2,17 (3H, s), 1,90 (2H, quint.), 1,83 (2H, quint.).

### 1.2 1-(4-{4-[2-tert-Butyl-6-(trifluormethyl)pyrimidin-4-yl]piperazin-1-yl}butyl)-4-methylpyridin-2(1H)-on

1-(4-Chlorbutyl)-4-methylpyridin-2(1*H*)-on (0,99 g, 4,96 mmol) aus Beispiel 1.1, 2-*tert-*Butyl-4-piperazin-1-yl-6-(trifluormethyl)pyrimidin (1,36 g, 4,71 mmol; hergestellt wie in DE 19735410 beschrieben) und Triethylamin (1,51 g, 14,87 mmol) in 25 ml Dimethylsulfoxid wurden 5 Stunden bei 100 °C gerührt. Danach versetzte man das Reaktionsgemisch mit Wasser und extrahierte das wässrige Gemisch zweimal mit tert.-Butyl-methylether. Die organische Phase extrahierte man dreimal mit einer gesättigten wässrigen KochsalzLösung und danach dreimal mit einer 5%igen wässrigen Zitronensäure-Lösung. Anschließend stellte man die wässrige Phase alkalisch und extrahierte sie dreimal mit *tert.-*Butyl-methylether. Die vereinten organischen Phasen trocknete man über Na₂SO₄, filtrierte das Trockenmittel ab und engte ein. Den erhaltenen öligen Rückstand (1,99 g) reinigte man durch Chromatographie an Kieselgel (Eluierungsmittel: CH₂Cl₂/CH₃OH: 96,5:3,5), wobei man 1,29 g der Titelverbindung erhielt.

¹H-NMR (500 MHz, CDCl₃) δ (ppm): 7,15 (1H, d), 6,59 (1H, s), 6,34 (1H, s), 6,00 (1H, d), 3,92 (2H, t), 3,70 (4H, s br.), 2,50 (4H, t), 2,41 (2H, t), 2,18 (3H, s), 1,80 (2H, quint.), 1,57 (2H, quint.), 1,33 (9H, s).

### Beispiel 2: 1-(4-{4-[2-tert-Butyl-6-(trifluormethyl)pyrimidin-4-yl]piperazin-1-yl}butyl)-5-(trifluormethyl)pyridin-2(1H)-on

### 2.1 1-(4-Chlorbutyl)-5-(trifluormethyl)pyridin-2(1H)-on

Analog zu Beispiel 1.1 erhielt man durch Umsetzung von 5-(Trifluormethyl)-2-pyridinol (1,63 g, 10 mmol) mit 1-Brom-4-chlorbutan 1,95 g der Titelverbindung.

ESI-MS: [M+H⁺] = 254,1.

### 2.2 1-(4-{4-[2-tert-Butyl-6-(trifluormethyl)pyrimidin-4-yl]piperazin-1-yl}butyl)-5-(trifluormethyl)pyridin-2(1H)-on

Analog zu Beispiel 1.2 erhielt man aus durch Umsetzung von 1-(4-Chlorbutyl)-5-(trifluormethyl)pyridin-2(1*H*)-on (0,65 g, 2,56 mmol) aus Beispiel 2.1 0,39 g der Titelverbindung.

¹H-NMR (500 MHz, CDCl₃) δ (ppm): 7,66 (1H, s), 7,45 (1H, d), 6,63 (1H, d), 6,58 (1H, s), 4,00 (2H, t), 3,73 (4H, s br.), 2,51 (4H, t), 2,43 (2H, t), 1,83 (2H, quint.), 1,60 (2H, quint.), 1,32 (9H, s).

### Beispiel 3: 4-(2-tert-Butyl-6-propylpyrimidin-4-yl)-1-[4-(4-methyl-2-oxopyridin-1 (2H)-yl)butyl]piperazin-1-iumchlorid

Analog zu Beispiel 1.2 erhielt man durch Umsetzung von 1-(4-Chlorbutyl)-4-methylpyridin-2(1*H*)-on (2,50 mmol, 0,50 g) aus Beispiel 1.1 mit 2-*tert*-Butyl-4-piperazin-1-yl-6-propylpyrimidin (0,62 g, 2,38 mmol; hergestellt wie in DE 19735410 beschrieben) 0,74 g der Titelverbindung.

ESI-MS: 427,5, [M+H⁺] = 426,5, 213,8.

### Beispiel 4: 4-(2-tert-Butyl-6-isopropylpyrimidin-4-yl)-1-[4-(4-methyl-2-oxopyridin-1(2H)-yl)butyl]piperazin-1-iumchlorid

Analog zu Beispiel 1.2 erhielt man durch Umsetzung von 1-(4-Chlorbutyl)-4-methylpyridin-2(1*H*)-on (1,25 mmol, 0,25 g) aus Beispiel 1.1 mit 2-*tert*-Butyl-4-piperazin-1-yl-6-isopropylpyrimidin (0,31 g, 1,19 mmol; hergestellt wie in DE 19735410 beschrieben) 0,38 g der Titelverbindung.

ESI-MS: 427,4, [M+H⁺] = 426,2, 213,8.

### Beispiel 5: 1-{4-[4-(2-tert-Butyl-6-propyl-pyrimidin-4-yl)-piperazin-1-yl]-butyl}-3-methoxy-1H-pyridin-2-on

### 5.1 1-(4-Chlorbutyl)-3-methoxy-1H-pyridin-2-on

Zu einer Suspension aus Natriumhydrid (20 mmol, 0,74 g, 60 %, entölt) in N,N-Dimethylformamid (100 ml) tropfte man bei 10 °C innerhalb 10 Minuten 3-Methoxy-1H-pyridin-2-on (20 mmol, 2,00 g) in 100 ml N,N-Dimethylformamid und rührte danach 1 Stunde bei Raumtemperatur nach. Anschließend tropfte man 1-Brom-4-chlorbutan (20 mmol, 3,19 g) in 40 ml N,N-Dimethylformamid zu. Das Reaktionsgemisch rührte man danach bei 95 °C nach. Nach dem Einengen des Reaktionsgemischs suspendierte man das verbliebene Öl in Diethylether. Die erhaltene Suspension wurde abfiltriert und das Filtrat dreimal mit Wasser und danach dreimal mit einer gesättigten wässrigen KochsalzLösung gewaschen. Nach dem Trocknen der organischen Phase über Natriumsulfat filtrierte man das Trockenmittel ab und engte ein. Der erhaltene Rückstand enthielt ein Gemisch aus O-alkylierter und N-alkylierter Verbindung. Die Chromatographie des Rückstandes an Kieselgel (Eluierungsmittel: CH₂Cl₂/CH₃OH: 0-2%) ergab 1,75 g der Titelverbindung.

¹H-NMR (400 MHz, CDCl₃) δ (ppm): 6,88 (1H, d), 6,60 (1H, d), 6,12 (1H, t), 4,02 (2H, t), 3,81 (3H, s), 3,57 (1H, t), 3,44 (1H, t), 2,02-1,72 (4H, m).

### 5.2 1-{4-[4-(2-tert-Butyl-6-propyl-pyrimidin-4-yl)-piperazin-1-yl]-butyl}-3-methoxy-1H-pyridin-2-on

Man erhitzte ein Gemisch aus 1-(4-Chlorbutyl)-3-methoxy-1H-pyridin-2-on (0,93 mmol, 0,20 g) aus Beispiel 5.1, 2-*tert*-Butyl-4-piperazin-1-yl-6-propylpyrimidin (0,93 mmol, 0,24 g; hergestellt wie in DE 19735410 beschrieben), Natriumbromid (4,64 mmol, 0,48 g), Ethyldiisopropylamin (9,09 mmol, 1,17 g) und N-Methylpyrrolidinon (0,5 ml) 6 Stunden auf 120°C. Die erhaltene Suspension saugte man ab und engte das Filtrat ein. Den auf diese Weise erhaltenen Rückstand nahm man in Ethylacetat/Wasser auf. Das wässrige Gemisch stellte man mit Natriumhydrogencarbonat auf pH 5,5 und extrahierte mehrmals das wässrige Gemisch mit Diethylether. Danach trocknete man die organische Phase, filtrierte das Trockenmittel ab und engte die organische Phase unter vermindertem Druck ein. Die Chromatographie des Rückstandes an Kieselgel (Eluierungsmittel: CH₂Cl₂/CH₃OH (0-2%) ergab 0,24 g der Titelverbindung.

ESI-MS: [M+H⁺] = 442,4, 221,6.

### Beispiel 6: 1-{4-[4-(2-tert-Butyl-6-trifluormethyl-pyrimidin-4-yl)-piperazin-1-yl]-butyl}-3-methoxy-1H-pyridin-2-on

Analog zu Beispiel 5.2 erhielt man durch Umsetzung von 1-(4-Chlorbutyl)-3-methoxy-1H-pyridin-2-on (0,93 mmol, 0,20 g) aus Beispiel 5.1 mit 2-*tert*-Butyl-4-piperazin-1-yl-6-(trifluormethyl)pyrimidin (0,93 mmol, 0,27g; hergestellt wie in DE 19735410 beschrieben) 0,25 g der Titelverbindung.

ESI-MS: [M+H⁺] = 468,2;

¹H-NMR (400 MHz, DMSO-d₆) δ (ppm): 7,27 (1H, d), 6,80 (1H, d), 6,15 (1H, t), 4,66 (2H, s br.), 3,90 (2H, t), 3,57-3,36 (4H, m), 3,17-2,95 (4H, m), 1,64 (4H, m sym.), 1,29 (9H, s).

### Beispiel 7: 1-{4-[4-(2-tert-Butyl-6-propyl-pyrimidin-4-yl)-piperazin-1-yl]-butyl}-3-methyl-1H-pyridin-2-on

### 7.1 1-(4-Chlorbutyl)-3-methyl-1H-pyridin-2-on

Analog zu Beispiel 1.1 erhielt man durch Umsetzung von 3-Methyl-1H-pyridin-2-on (17,96 mmol, 2,00 g) mit 1-Brom-4-chlorbutan 1,98 g der Titelverbindung.

ESI-MS: [M+H⁺] = 200,05;

¹H-NMR (400 MHz, CDCl₃) δ (ppm): 7,19 (1H, d), 7,13 (1H, d), 6,10 (1H, t), 3,99 (2H, t), 3,58 (2H, t), 2,16 (3H, s), 2,05-1,75 (4H, m).

### 7.2 1-{4-[4-(2-tert-Butyl-6-propyl-pyrimidin-4-yl)-piperazin-1-yl]-butyl}-3-methyl-1H-pyridin-2-on

Analog zu Beispiel 5.2 erhielt man durch Umsetzung von 1-(4-Chlorbutyl)-3-methyl-1H-pyridin-2-on (1,00 mmol, 0,20 g) aus Beispiel 7.1 mit 2-*tert*-Butyl-4-piperazin-1-yl-6-propylpyrimidin (1,00 mmol, 0,26 g; hergestellt wie in DE 19735410 beschrieben) 0,19 g der Titelverbindung.

ESI-MS: [M+H⁺] = 426,4, 213,8.

### Beispiel 8: 4-(2-tert-Butyl-6-propyl-pyrimidin-4-yl)-1-[4-(4-chlor-2-oxo-2H-pyridin-1-yl)-butyl]-piperazin als Hydrochlorid

### 8.1 4-Chlor-1-(4-chlorbutyl)-1H-pyridin-2-on

Analog zu Beispiel 1.1 erhielt man durch Umsetzung von 4-Chlorpyridin-2-ol (1,54 mmol, 0,20 g) mit 1-Brom-4-chlorbutan 0,20 g der Titelverbindung.

¹H-NMR (400 MHz, CDCl₃) δ (ppm): 7,20 (1H, d), 6,60 (1H, s), 6,20 (1H, d), 3,94 (2H, t), 3,58 (2H, t), 1,90 (2H, quint.), 1,81 (2H, quint.).

### 8.2 4-(2-tert-Butyl-6-propyl-pyrimidin-4-yl)-1-[4-(4-chlor-2-oxo-2H-pyridin-1-yl)-butyl]-piperazin-Hydrochlorid

Analog zu Beispiel 5.2 erhielt man durch Umsetzung von 4-Chlor-1-(4-chlorbutyl)-1H-pyridin-2-on (0,45 mmol, 0,10 g) aus Beispiel 8.1 mit 2-*tert*-Butyl-4-piperazin-1-yl-6-propylpyrimidin (0,43 mmol, 0,11g; hergestellt wie in DE 19735410 beschrieben) 0,16 g der Titelverbindung.

ESI-MS: 448,2, 446,3, 224,6, 223,6;

¹H-NMR (400 MHz, CDCl₃) δ (ppm): 7,20 (1H, d), 6,60 (1H, s), 6,18 (1H, d), 6,12 (1H, s), 3,95 (2H, t), 3,60 (4H, s br.), 2,60-2,33 (8H, m einschließlich 2,53 (2H, t), 2,40 (2H, t)), 1,83-1,49 (6H, m), 1,33 (9H, s), 0,97 (3H, t).

### Beispiel 9: 4-(2-tert-Butyl-6-trifluormethyl-pyrimidin-4-yl)-1-[4-(4-chlor-2-oxo-2H-pyridin-1-yl)-butyl]-piperazin als Hydrochlorid

Analog zu Beispiel 5.2 erhielt man durch Umsetzung von 4-Chlor-1-(4-chlorbutyl)-1H-pyridin-2-on (0,45 mmol, 0,10 g) aus Beispiel 8.1 mit 2-*tert*-Butyl-4-piperazin-1-yl-6-(trifluormethyl)pyrimidin (0,43 mmol, 0,11 g; hergestellt wie in DE 19735410 beschrieben) 0,12 g der Titelverbindung.

ESI-MS: 474,2, 472,2, 237,4, 236,6;

¹H-NMR (400 MHz, CDCl₃) δ (ppm): 7,20 (1H, d), 6,60 (1H, s), 6,58 (1H, s), 6,19 (1H, d), 3,94 (2H, t), 3,68 (4H, s br.), 2,47 (2H, t), 2,39 (2H, t), 1,77 (2H, quint.), 1,65 (2H+H₂O, quint.), 1,33 (9H, s).

### Beispiel 10: 1-{4-[4-(2-tert-Butyl-6-propyl-pyrimidin-4-yl)-piperazin-1-yl]-butyl}-4-hydroxy-1H-pyridin-2-on

### 10.1 1-(4-Chlorbutyl)-4-hydroxy-1H-pyridin-2-on

Analog zu Beispiel 1.1 erhielt man durch Umsetzung von 4-Hydroxy-1H-pyridin-2-on (18,00 mmol, 2,00 g) mit 1-Brom-4-chlorbutan 1,30 g der Titelverbindung.

### 10.2 1-{4-[4-(2-tert-Butyl-6-propyl-pyrimidin-4-yl)-piperazin-1-yl]-butyl}-4-hydroxy-1H-pyridin-2-on

Analog zu Beispiel 5.2 erhielt man durch Umsetzung von 1-(4-Chlorbutyl)-4-hydroxy-1H-pyridin-2-on (2,48 mmol, 0,50 g) aus Beispiel 10.1 mit 2-*tert*-Butyl-4-piperazin-1-yl-6-propylpyrimidin (2,48 mmol, 0,65 g; hergestellt wie in DE 19735410 beschrieben) 0,40 g der Titelverbindung.

ESI-MS: [M+H⁺] = 428,4, 214,6;

¹H-NMR (400 MHz, DMSO-d₆) δ (ppm): 11,93 (1H, s br.), 7,17 (1H, d), 6,12 (1H, s), 5,95 (1H, d), 5,87 (1H, s), 3,97 (2H, t), 3,62 (4H, s br.), 2,63-2,36 (8H, m), 1,81 (2H, quint.), 1,66 (4H+H₂O, quint.), 1,33 (9H, s), 0,96 (3H, t).

### Beispiel 11:1-{4-[4-(2-tert-Butyl-6-trifluormethyl-pyrimidin-4-yl)-piperazin-1-yl]-butyl}-3-methyl-1H-pyridin-2-on

Analog zu Beispiel 5.2 erhielt man durch Umsetzung von 1-(4-Chlorbutyl)-3-methyl-1H-pyridin-2-on (1,50 mmol, 0,30 g) aus Beispiel 7.1 mit 2-*tert*-Butyl-4-piperazin-1-yl-6-(trifluormethyl)pyrimidin (1,53 mmol, 0,44 g; hergestellt wie in DE 19735410 beschrieben) 0,34 g der Titelverbindung.

ESI-MS: [M+H⁺] = 452,2, 226,6;

¹H-NMR (400 MHz, DMSO-d₆) δ (ppm): 7,50 (1H, d), 7,27 (1H, d), 7,03 (1H, s), 6,11 (1H, t), 3,87 (2H, t), 3,68 (4H, s br.), 2,57-2,35 (6H, m einschließlich 2,36 (2H, t)), 1,98 (3H, s), 1,62 (2H, quint.), 1,43 (2H, quint.), 1,25 (9H, s).

### Beispiel 12:1-{4-[4-(2-tert-Butyl-6-trifluormethyl-pyrimidin-4-yl)-piperazin-1-yl]-butyl}-4-hydroxy-1H-pyridin-2-on

Analog zu Beispiel 5.2 erhielt man durch Umsetzung von 1-(4-Chlorbutyl)-4-hydroxy-1H-pyridin-2-on (1,24 mmol, 0,25 g) aus Beispiel 10.1 mit 2-tert-Butyl-4-piperazin-1-yl-6-(trifluormethyl)pyrimidin (1,24 mmol, 0,36 g; hergestellt wie in DE 19735410 beschrieben) 0,30 g der Titelverbindung.

ESI-MS: [M+H⁺] = 454,2, 227,6;

¹H-NMR (400 MHz, DMSO-d₆) δ (ppm): 12,04 (1H, s br.), 7,17 (1H, d), 6,58 (1H, s), 5,95 (1H, d), 5,86 (1H, s), 3,99 (2H, t), 3,71 (4H, s br.), 2,52 (4H, s br.), 2,43 (2H, t), 1,82 (2H, quint.), 1,77-1,51 (2H+H₂O, m), 1,35 (9H, s).

### Beispiel 13: 1-{4-[4-(2-tert-Butyl-6-trifluormethyl-pyrimidin-4-yl)-piperazin-1-yl]-butyl}-3-trifluormethyl-1H-pyridin-2-on

### 13.1 1-(4-Chlorbutyl)-3-trifluormethyl-1H-pyridin-2-on

Analog zu Beispiel 1.1 erhielt man durch Umsetzung von 3-Trifluormethyl-1H-pyridin-2-on (6,13 mmol, 1,00 g) mit 1-Brom-4-chlorbutan 1,10 g der Titelverbindung.

ESI-MS (N-Alk.): [M+H⁺] = 254,1;

¹H-NMR (400 MHz, CDCl₃) δ (ppm): 7,73 (1H, d), 7,47 (1H, d), 6,24 (1H, t), 4,03 (2H, t), 3,60 (2H, t), 1,95 (2H, q), 1,82 (2H, q).

ESI-MS (O-Alk.): [M+Na⁺] = 276,1, 256,1, [M+H⁺] = 254,1.

### 13.2 1-{4-[4-(2-tert-Butyl-6-trifluormethyl-pyrimidin-4-yl)-piperazin-1-yl]-butyl}-3-trifluormethyl-1H-pyridin-2-on

Analog zu Beispiel 5.2 erhielt man durch Umsetzung von 1-(4-Chlorbutyl)-3-trifluormethyl-1H-pyridin-2-on (0,59 mmol, 0,15 g) aus Beispiel 13.1 mit 2-*tert*-Butyl-4-piperazin-1-yl-6-(trifluormethyl)pyrimidin (0,59 mmol, 0,17 g; hergestellt wie in DE 19735410 beschrieben) 0,17 g der Titelverbindung.

ESI-MS: [M+H⁺] = 506,2, 253,6;

¹H-NMR (400 MHz, CDCl₃) δ (ppm): 7,75 (1H, d), 7,50 (1H, d), 6,68 (1H, s), 6,22 (1H, t), 4,01 (2H, t), 3,68 (4H, s br.), 2,51 (4H, s br.), 2,43 (2H, t), 1,84 (2H, quint.), 1,72-1,46 (2H+H₂O, s br.), 1,33 (9H, s).

### Beispiel 14: 1-{4-[4-(2-tert-Butyl-6-propyl-pyrimidin-4-yl)-piperazin-1-yl]-butyl}-3-trifluormethyl-1H-pyridin-2-on

Analog zu Beispiel 5.2 erhielt man durch Umsetzung von 1-(4-Chlorbutyl)-3-trifluormethyl-1H-pyridin-2-on (0,59 mmol, 0,15 g) aus Beispiel 13.1 mit 2-*tert*-Butyl-4-piperazin-1-yl-6-propylpyrimidin (0,59 mmol, 0,16 g; hergestellt wie in DE 19735410 beschrieben) 0,15 g der Titelverbindung.

ESI-MS: [M+H⁺] = 480,2, 240,6;

¹H-NMR (400 MHz, DMSO-d₆) δ (ppm): 8,04 (1H, d), 7,91 (1H, d), 6,43 (1H, s), 6,35 (1H, t), 3,97 (2H, t), 3,57 (4H, s br.), 2,56-2,27 (6H, m einschließlich 2,33 (2H, t)), 1,74-1,55 (4H, m), 1,45 (2H, quint.), 1,25 (9H, s), 0,90 (3H, t).

### Beispiel 15: 4-(2-tert-Butyl-6-propyl-pyrimidin-4-yl)-1-[4-(2-oxo-4-trifluormethyl-2H-pyridin-1-yl)-butyl]-piperazin als Fumarat

### 15.1 1-(4-Chlorbutyl)-4-trifluormethyl-1H-pyridin-2-on

Analog zur Herstellungsvorschrift aus Beispiel 1.1 erhielt man durch Umsetzung von 4-Trifluoromethyl-1H-pyridin-2-one (3,07 mmol, 0,50 g) mit 1-Brom-4-chlorbutan die Titelverbindung in einer Ausbeute von 0,45 g.

### 15.2 4-(2-tert-Butyl-6-propyl-pyrimidin-4-yl)-1-[4-(2-oxo-4-trifluormethyl-2H-pyridin-1-yl)-butyl]-piperazin als Fumarat

Analog zur Herstellungsvorschrift aus Beispiel 5.2 erhielt man durch Umsetzung von 1-(4-Chlorbutyl)-4-trifluormethyl-1H-pyridin-2-on (0,63 mmol, 0,16g) mit 2-tert-Butyl-4-piperazin-1-yl-6-propylpyrimidin (0,60 mmol, 0,16g, Herstellung nach DE 19735410) die Titleverbindung in einer Ausbeute von 0,24 g.

ESI-MS: [M+H⁺] = 480,25, 240,65;

¹H-NMR (400 MHz, DMSO-d₆) δ (ppm): 7,96 (1H, d), 6,76 (1H, s), 6,47 (1H, m), 6,44 (1H, s), 3,96 (2H, t), 3,56 (4H, s), 2,58-2,23 (8H, m), 1,74-1,38 (6H, m), 1,25 (9H, s), 0,89 (3H, t).

### Beispiel 16: 4-(2-tert-Butyl-6-trifluormethylpyrimidin-4-yl)-1-[4-(2-oxo-4-trifluormethyl-2H-pyridin-1-yl)-butyl]-piperazin als Fumarat

Analog zu der Herstellungsvorschrift in Beispiel 1.2 erhielt man durch Umsetzung von 1-(4-Chlorbutyl)-4-trifluoromethyl-1H-pyridin-2-on (0,63 mmol, 0,16 g) mit 2-tert-Butyl-4-piperazin-1-yl-6-trifluormethylpyrimidin (0,60 mmol, 0,17 g, Herstellung nach DE 19735410) die Titelverbindung in einer Ausbeute von 0,12 g.

ESI-MS: [M+Na⁺] = 528,2, 507,2, [M+H⁺] = 506,1, 253,6;

### Beispiel 17: 4-(2-tert-Butyl-6-propyl-pyrimidin-4-yl)-1-[4-(5-chloro-2-oxo-2H-pyridin-1-yl)-butyl]-piperazin als Fumarat

### 17.1: 5-Chlor-1-(4-chlorbutyl)-1H-pyridin-2-on

Analog zu Beispiel 1.1 erhielt man durch Umsetzung von 5-Chlor-1H-pyridin-2-on (15,44 mmol, 2,00 g) mit 1-Brom-4-chlorbutan 1,63 g der Titelverbindung.

ESI-MS: [M+H⁺] = 221,9, 220,9, 219,9;

### 17.2 4-(2-tert-Butyl-6-propyl-pyrimidin-4-yl)-1-[4-(5-chloro-2-oxo-2H-pyridin-1-yl)-butyl]-piperazin als Fumarat

Analog zur Vorschrift aus Beispiel 1.2 erhielt man durch Umsetzung von 5-Chlor-1-(4-chlor-butyl)-1H-pyridin-2-on (0,91 mmol, 0,20 g) mit 2-tert-Butyl-4-piperazin-1-yl-6-propylpyrimidin (0,82 mmol, 0,21 g, Herstellung nach DE 19735410) in einer Ausbeute von 0,35g.

ESI-MS: 448,2, [M+H⁺] = 446,3, 244,4, 223,6;

¹H-NMR (400 MHz, DMSO-d₆) δ (ppm): 7,96 (1H, d), 7,46 (1H, dd), 6,46-6,35 (2H, m), 3,86 (2H, t), 3,58 (4H, s br.), 2,45 (6H, s br.), 1,63 (4H, sext.), 1,43 (2H, quint.), 1,24 (9H, s), 0,90 (3H, t).

### Beispiel 18: 4-(2-Tert-butyl-6-trifluormethylpyrimidin-4-yl)-1-[4-(5-chlor-2-oxo-2H-pyridin-1-yl)-butyl]-piperazin als Fumarat

Analog zur Vorschrift aus Beispiel 1.2 erhielt man durch Umsetzung von 5-Chlor-1-(4-chlor-butyl)-1H-pyridin-2-on (0,91 mmol, 0,20 g) und 2-tert-Butyl-4-piperazin-1-yl-6-trifluormethyl-pyrimidin (0,82 mmol, 0,24 g, Herstellung nach DE 19735410 die Titelverbindung in einer Ausbeute von 0,23 g.

ESI-MS: 474,1, [M+H⁺] = 472,1, 237,4, 236,6;

¹H-NMR (500 MHz, DMSO-d₆) δ (ppm): 7,97 (1H, s), 7,44 (1H, d), 7,04 (1H, s), 6,41 (1H, d), 3,86 (2H, t), 3,70 (4H, s br.), 2,44 (4H, m sym.), 2,34 (2H, t), 1,65 (2H, quint.), 1,44 (2H, quint.), 1,28 (9H, s).

### Beispiel 19:1-{4-[4-(2-tert-Butyl-6-trifluoromethyl-pyrimidin-4-yl)-piperazin-1-yl]-butyl}-4-phenyl-1H-pyridin-2-on

### 19.1: 1-(4-Chlorbutyl)-4-phenyl-1H-pyridin-2-on

Analog zu Beispiel 1.1 erhielt man durch Umsetzung von 4-Phenyl-1H-pyridin-2-on (0,41 mmol, 71,0 mg, hergestellt nach Tetrahedron 1997, 53, S.14437-50, aus 4-Chlor-1H-pyridin-2-on) mit 1-Brom-4-chlorbutan 34 mg der Titelverbindung.

ESI-MS: 202,1,[M+H⁺] = 200,1;

### 19.2: 1-{4-[4-(2-tert-Butyl-6-trifluormethyl-pyrimidin-4-yl)-piperazin-1-yl]-butyl}-4-phenyl-1H-pyridin-2-on

Analog zur Vorschrift aus Beispiel 1.2 erhält man durch Umsetzung von 1-(4-Chlorbutyl)-4-phenyl-1H-pyridin-2-on (0,13 mmol, 34,0 mg) mit 2-tert-Butyl-4-piperazin-1-yl-trifluormethylpyrimidine (0,13 mmol, 37,5 mg, Herstellung nach DE 19735410) die Titelverbindung in einer Ausbeute von 12 mg.

ESI-MS: [M+Na⁺] = 536,2, 515,2,[M+H⁺] = 514,2, 257,6;

¹H-NMR (400 MHz, DMSO-d₆) δ (ppm): 7,77 (1H, d), 7,72 (2H, d), 7,53-7,41 (3H, m), 7,03 (1H, s br.), 6,66 (1H, s), 6,58 (1H, d), 3,93 (2H, t), 3,70 (4H, s br.), 2,41 (4H, s br.), 2,33 (2H, m), 1,68 (2H, quint.), 1,48 (2H, m), 1,27 (9H, s).

### Beispiel 20: 4-(2-tert-Butyl-6-propylpyrimidin-4-yl)-1-[4-(6-methyl-2-oxo-2H-pyridin-1-yl)-butyl]-piperazin als Fumarat

### 20.1 1-(4-Chloro-butyl)-6-methyl-1H-pyridin-2-on

Analog zu Beispiel 1.1 erhielt man durch Umsetzung von 6-Methyl-1H-pyridin-2-on (18,33 mmol, 2,00 g) mit 1-Brom-4-chlorbutan 0,40 g der Titelverbindung.

ESI-MS: 202,1,[M+H⁺] = 200,1;

### 20.2 4-(2-tert-Butyl-6-propylpyrimidin-4-yl)-1-[4-(6-methyl-2-oxo-2H-pyridin-1-yl)-butyl]-piperazin als Fumarat

Analog zur Vorschrift aus Beispiel 1.2 erhält man durch Umsetzung von 1-(4-Chlorbutyl)-6-methyl-1H-pyridin-2-on (0,75 mmol, 0,15 mg) mit 2-tert-Butyl-4-piperazin-1-yl-6-propylpyrimidin (0,67 mmol, 0,18 g, Herstellung nach DE 19735410) die Titelverbindung in einer Ausbeute von 0,18 g.

ESI-MS: 427,4,[M+H⁺] = 426,4, 213,6;

¹H-NMR (500 MHz, DMSO-d₆) δ (ppm): 7,24 (1H, m sym.), 6,43 (1H, s), 6,21 (1H, d), 6,06 (1H, d), 3,96 (2H, t), 3,59 (4H, s br.), 2,47 (8H, m), 2,37 (3H, s), 1,66-1,49 (6H, m), 1,27 (9H, s), 0,90 (3H, t).

### Beispiel 21: 4-(2-tert-Butyl-6-trifluoromethyl-pyrimidin-4-yl)-1-[4-(6-methyl-2-oxo-2H-pyridin-1-yl)-butyl]-piperazin als Fumarat

Analog zur Vorschrift aus Beispiel 1.2 erhält man durch Umsetzung von 1-(4-Chlorobutyl)-6-methyl-1H-pyridin-2-on 0,75 mmol, 0,15 mg) mit 2-tert-Butyl-4-piperazin-1-yl-6-trifluoromethyl-pyrimidin (0,68 mmol, 0,19 g, Herstellung nach DE 19735410) die Titelverbindung in einer Ausbeute von 0,24 g.

ESI-MS: [M+H⁺] = 452,2, 226,6;

¹H-NMR (500 MHz, DMSO-d₆) δ (ppm): 7,23 (1H, m sym.), 7,03 (1H, s), 6,21 (1H, d), 6,08 (1H, d), 3,96 (2H, t), 3,72 (4H, s br.), 2,46 (4H, m), 2,41-2,34 (5H, m), 1,59 (2H, quint.), 1,52 (2H, quint.), 1,28 (9H, s).

### II Beispiele für galenische Applikationsformen

### Tabletten:

Auf einer Tablettenpresse werden in üblicher Weise Tabletten folgender Zusammensetzung gepresst:
40 mg Substanz des Beispiels 2
120 mg Maisstärke
13,5 mg Gelatine
45 mg Milchzucker
2,25 mg Aerosil® (chemisch reine Kieselsäure in submikroskopisch feiner Verteilung)
6,75 mg Kartoffelstärke (als 6%iger Kleister)

### Dragees:

20 mg Substanz des Beispiels 2
60 mg Kernmasse
70 mg Verzuckerungsmasse

Die Kernmasse besteht aus 9 Teilen Maisstärke, 3 Teilen Milchzucker und 1 Teil Vinylpyrrolidon-Vinylacetat-Mischpolymerisat 60:40. Die Verzuckerungsmasse besteht aus 5 Teilen Rohrzucker, 2 Teilen Maisstärke, 2 Teilen Calciumcarbonat und 1 Teil Talk. Die so hergestellten Dragees werden anschließend mit einem magensaftresistenten Überzug versehen.

### III. Biologische Untersuchungen - Rezeptorbindungsstudien:

Die zu testende Substanz wurde entweder in Methanol/Chremophor® (BASF-AG) oder in Dimethylsulfoxid gelöst und anschließend mit Wasser auf die gewünschte Konzentration verdünnt.

### III.1 Dopamin-D₃-Rezeptor:

Der Ansatz (0,250 ml) setzte sich zusammen aus Membranen von ∼ 10⁶ HEK-293 Zellen mit stabil exprimierten humanen Dopamin-D₃-Rezeptoren, 0,1 nM [¹²⁵I]-Jodosulpirid und Inkubationspuffer (totale Bindung) oder zusätzlich Testsubstanz (Hemmkurve) oder 1µM Spiperon (unspezifische Bindung). Dreifach-Ansätze wurden durchgeführt.

Der Inkubationspuffer enthielt 50 mM Tris, 120 mM NaCl, 5 mM KCI, 2 mM CaCl₂, 2 mM MgCl₂ und 0,1% Rinderserumalbumin, 10 µM Quinolone, 0.1 % Ascorbinsäure (täglich frisch hergestellt). Der Puffer wurde mit HCl auf pH 7,4 eingestellt.

### III.2 Dopamin-D_{2L}-Rezeptor:

Der Ansatz (1 ml) setzte sich zusammen aus Membranen von - 10⁶ HEK-293 Zellen mit stabil exprimierten humanen Dopamin-D_{2L} Rezeptoren (lange Isoform) sowie 0,01 nM [¹²⁵I]-Jodospiperon und Inkubationspuffer (totale Bindung) oder zusätzlich Testsubstanz (Hemmkurve) oder 1µM Haloperidol (unspezifische Bindung). Dreifach-Ansätze wurden durchgeführt.

Der Inkubationspuffer enthielt 50 mM Tris, 120 mM NaCl, 5 mM KCI, 2 mM CaCl₂, 2 mM MgCl₂ und 0, 1 % Rinderserumalbumin. Der Puffer wurde mit HCl auf pH 7,4 eingestellt.

### III.3 Messung und Auswertung:

Nach Inkubation für 60 Minuten bei 25 °C wurden die Ansätze mit einem Zellsammelgerät über Whatman GF/B Glasfaserfilter unter Vakuum filtriert. Die Filter wurden mit einem Filter-Transfer-System in Szintillationsgläser überführt. Nach Zugabe von 4 ml Ultima Gold^{®} (Packard) wurden die Proben eine Stunde geschüttelt und anschließend die Radioaktivität im Beta-Counter (Packard, Tricarb 2000 oder 2200CA) gezählt. Die cp-Werte wurden anhand einer Standard-Quenchreihe mit Hilfe des geräteeigenen Programms in dpm umgerechnet.

Die Auswertung der Hemmkurven erfolgte durch iterative nichtlineare Regressionsanalyse mit dem Statistical Analysis System (SAS), ähnlich dem von Munson und Rodbard beschreibenen Programm "LIGAND".

Die erfindungsgemäßen Verbindungen zeigen in diesen Tests sehr gute Affinitäten am D₃-Rezeptor (< 100 nM, häufig < 50 nM, insbesondere > 10 nM) und binden selektiv an den D₃-Rezeptor.

Die Ergebnisse der Bindungstests sind in Tabelle 2 angegeben.

**Tabelle 2:**

| Beispiel | Kᵢ (D₃) [nM] | Selektivität vs. D₂L |
|---|---|---|
| 1 | 0,76 | 82 |
| 3 | 0,84 | 137 |
| 5 | 1,20 | 51 |
| 6 | 2,20 | 74 |
| 7 | 1,25 | 129 |
| 11 | 2,31 | 74 |
| 19 | 7,89 | 63 |

| | | |
|---|---|---|
| * Kᵢ(D_{2L})/Kᵢ(D₃) | | |

## Patentansprüche

1. Pyridin-2-onverbindungen der allgemeinen Formel I 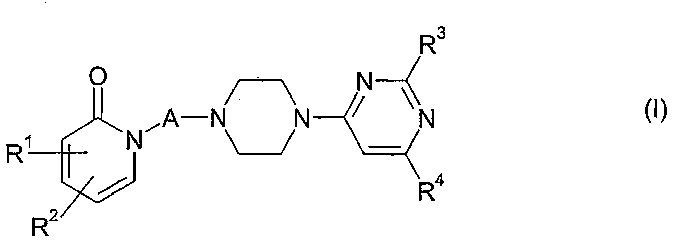 worin
A für eine 4- bis 6-gliedrige Kohlenwasserstoffkette steht, die 1 oder 2 Methylgruppen als Substituenten aufweisen kann, worin 1 oder 2 Kohlenstoffatome durch Sauerstoff, eine Carbonylgruppe oder Schwefel ersetzt sein können, und worin die Kohlenwasserstoffkette eine Doppelbindung oder eine Dreifachbindung aufweisen kann;
R¹, R² unabhängig voneinander für Wasserstoff, CN, NO₂, Halogen OR⁵, NR⁶R⁷, C(O)NR⁶R⁷, O-C(O)NR⁶R⁷, SR⁸, SOR⁸, SO₂R⁸, SO₂NR⁶R⁷, COOR⁹, O-C(O)R¹⁰, COR¹⁰, C₁-C₆-Alkyl, C₁-C₆-Halogenalkyl, C₂-C₆-Alkenyl, C₂-C₆-Alkinyl, C₂-C₆-Halogenalkenyl, C₃-C₆-Cycloalkyl, 4- bis 6-gliedriges Heterocyclyl mit 1, 2 oder 3 Heteroatomen, ausgewählt unter O, S und N, das 1, 2 oder 3 Substituenten aufweisen kann, die unabhängig voneinander ausgewählt sind unter C₁-C₄-Alkyl, C₁-C₄-Alkoxy, NR⁶R⁷, CN, OH, C₁-C₂-Fluoralkyl oder Halogen, Phenyl, das 1, 2 oder 3 Substituenten aufweisen kann, die unabhängig voneinander ausgewählt sind unter C₁-C₄-Alkyl, C₁-C₄-Alkoxy, NR⁶R⁷, OH, CN, C₁-C₂-Fluoralkyl oder Halogen, C₁-C₆-Alkyl, das einen Substituenten aufweist, der ausgewählt ist unter OR⁵, NR⁶R⁷, C(O)NR⁶R⁷, O-C(O)NR⁶R⁷, SR⁸, SOR⁸, SO₂R⁸, SO₂NR⁶R⁷, COOR⁹, O-C(O)R¹⁰, COR¹⁰, C₃-C₆-Cycloalkyl, 5- oder 6-gliedrigem Heterocyclyl mit 1, 2 oder 3 Heteroatomen, ausgewählt unter O, S und N, und Phenyl, wobei Phenyl und Heterocyclyl 1, 2 oder 3 Substituenten aufweisen können, die unabhängig voneinander ausgewählt sind unter C₁-C₄-Alkyl, C₁-C₄-Alkoxy, NR⁶R⁷, CN, OH, C₁-C₂-Fluoralkyl oder Halogen, C₂-C₆-Alkenyl, das einen Substituenten, ausgewählt unter OR⁵, NR⁶R⁷, C(O)NR⁶R⁷, O-C(O)NR⁶R⁷, SR⁸, SOR⁸, SO₂R⁸, SO₂NR⁶R⁷, COOR⁹, O-C(O)R¹⁰, COR¹⁰, C₃-C₆-Cycloalkyl, 5- oder 6-gliedriges Heterocyclyl mit 1, 2 oder 3 Heteroatomen, ausgewählt unter O, S und N, und Phenyl aufweist, wobei Phenyl und Heterocyclyl ihrerseits 1, 2 oder 3 Substituenten aufweisen können, die unabhängig voneinander ausgewählt sind unter C₁-C₄-Alkyl, C₁-C₄-Alkoxy, NR⁶R⁷, OH, CN, C₁-C₂-Fluoralkyl oder Halogen, stehen;
R³, R⁴ unabhängig voneinander für OR⁵, NR⁶R⁷, CN, C₁-C₆-Alkyl, das gegebenenfalls durch OH, C₁-C₄-Alkoxy, Halogen oder Phenyl ein- oder mehrfach substituiert ist, das seinerseits 1, 2 oder 3 Substituenten, ausgewählt unter C₁-C₄-Alkyl, C₁-C₄-Alkoxy, NR⁶R⁷, OH, CN, C₁-C₂-Fluoralkyl oder Halogen, tragen kann, C₂-C₆-Alkenyl, C₂-C₆-Alkinyl, C₃-C₆-Cycloalkyl, C₄-C₁₀-Bicycloalkyl, C₆-C₁₀-Tricycloalkyl, wobei die 5 letztgenannten Gruppen gegebenenfalls ein- oder mehrfach mit Halogen oder C₁-C₄-Alkyl substituiert sein können, Halogen, CN, C₁-C₄-Alkoxy, 5- oder 6-gliedriges Heterocyclyl mit 1, 2 oder 3 Heteroatomen, ausgewählt unter O, S und N, und Phenyl, wobei Phenyl und Heterocyclyl gegebenenfalls 1, 2 oder 3 Substituenten tragen, die unabhängig voneinander ausgewählt sind unter C₁-C₄-Alkyl, C₁-C₄-Alkoxy, NR⁶R⁷, CN, C₁-C₂-Fluoralkyl und Halogen, stehen;
R⁵, R⁶, R⁷ R⁸, R⁹ und R¹⁰ unabhängig voneinander für H, C₁-C₆-Alkyl, das gegebenenfalls durch OH, C₁-C₄-Alkoxy oder Phenyl substituiert ist, das seinerseits 1, 2 oder 3 Substituenten, ausgewählt unter C₁-C₄-Alkyl, C₁-C₄-Alkoxy, NR⁶R⁷, OH, CN, C₁-C₂- , Fluoralkyl oder Halogen, tragen kann, C₁-C₆-Halogenalkyl oder Phenyl, das seinerseits 1, 2 oder 3 Substituenten, ausgewählt unter C₁-C₄-Alkyl, C₁-C₄-Alkoxy, NR⁶R⁷, OH, CN, C₁-C₂-Fluoralkyl oder Halogen, tragen kann, stehen, wobei
R⁷ auch eine Gruppe COR¹¹ bedeuten kann, und wobei
R⁶ mit R⁷ auch gemeinsam mit dem Stickstoffatom, an das sie gebunden sind, einen 4-, 5- oder 6-gliedrigen, gesättigten oder ungesättigten Heterocyclus bilden können, der ein weiteres Heteroatom, ausgewählt unter O, S und NR¹² als Ringglied aufweisen kann, wobei R¹² für Wasserstoff oder C₁-C₄-Alkyl steht, und der durch 1, 2, 3 oder 4 Alkylgruppen substituiert sein kann; und
R¹¹ für Wasserstoff, C₁-C₄-Alkyl oder Phenyl, das gegebenenfalls durch 1, 2 oder 3 Reste substituiert ist, die unabhängig voneinander ausgewählt sind unter C₁-C₄-Alkyl, C₁-C₄-Alkoxy, NR⁶R⁷, CN, C₁-C₂-Fluoralkyl oder Halogen, steht;
sowie die Tautomere der Verbindungen I, die physiologisch akzeptablen Salze der Verbindungen I und die physiologisch akzeptablen Salze der Tautomere der Verbindungen I.

2. Pyridin-2-onverbindungen nach Anspruch 1, worin R³ für C₁-C₆-Alkyl steht und R⁴ ausgewählt ist unter C₁-C₆-Alkyl, C₃-C₆-Cycloalkyl, das gegebenenfalls 1 oder 2 Substituenten, ausgewählt unter Chlor und Methyl aufweist, und C₁-C₂-Fluoralkyl.

3. Pyridin-2-onverbindungen nach Anspruch 2, worin R³ für verzweigtes Alkyl mit 3, 4 oder 5 C-Atomen oder für C₃-C₆-Cycloalkyl steht.

4. Pyridin-2-onverbindungen nach Anspruch 2 oder 3, worin R⁴ für Trifluormethyl oder C₃-C₄-Alkyl steht.

5. Pyridin-2-onverbindungen nach Anspruch 2 oder 3, worin R⁴ für Cyclopropyl, Cyclobutyl, Cyclopentyl oder 1-Methylcyclopropyl steht.

6. Pyridin-2-onverbindungen nach einem der vorhergehenden Ansprüche, worin wenigstens einer der Reste R¹ oder R² von Wasserstoff verschieden ist.

7. Pyridin-2-onverbindungen nach Anspruch 6, worin R¹ ausgewählt ist unter Halogen, OR⁵, NR⁶R⁷, C₁-C₄-Alkyl, das gegebenenfalls durch OH, C₁-C₄-Alkoxy oder Halogen substituiert ist, aromatischem, 5- oder 6-gliedriges Heterocyclyl mit 1, 2 oder 3 Heteroatomen, ausgewählt unter O, S und N, das 1, 2 oder 3 Substituenten aufweisen kann, die unabhängig voneinander ausgewählt sind unter C₁-C₄-Alkyl, C₁-C₄-Alkoxy, NR⁶R⁷, CN, OH, C₁-C₂-Fluoralkyl oder Halogen, und Phenyl, das 1, 2 oder 3 Substituenten aufweisen kann, die unabhängig voneinander ausgewählt sind unter C₁-C₄-Alkyl, C₁-C₄-Alkoxy, NR⁶R⁷, OH, CN, C₁-C₂-Fluoralkyl oder Halogen.

8. Pyridin-2-onverbindungen nach Anspruch 7, worin R¹ ausgewählt ist unter Phenyl, OH, Chlor, Methyl, Methoxy und Trifluormethyl.

9. Pyridin-2-onverbindungen nach einem der vorhergehenden Ansprüche, worin R² Wasserstoff bedeutet.

10. Pyridin-2-onverbindungen nach einem der vorhergehenden Ansprüche, worin A für Butan-1,4-diyl steht.

11. Pharmazeutisches Mittel, enthaltend wenigstens eine Verbindung nach einem der Ansprüche 1 bis 10 und/oder deren Salz, gegebenenfalls zusammen mit physiologisch akzeptablen Trägern und/oder Hilfsstoffen.

12. Verwendung von Verbindungen nach einem der Ansprüche 1 bis 10 und von deren pharmakologisch akzeptablen Salzen zur Herstellung eines pharmazeutischen Mittels zur Behandlung von Erkrankungen, die auf die Beeinflussung durch Dopamin-D₃-Rezeptorliganden ansprechen.

13. Verwendung von Verbindungen nach einem der Ansprüche 1 bis 10 und von deren pharmakologisch akzeptablen Salzen zur Herstellung eines pharmazeutischen Mittels zur Behandlung von Erkrankungen des zentralen Nervensystems.

14. Verwendung nach Anspruch 13 zur Behandlung von Schizophrenie und/oder Depression.

## Claims

1. A pyridin-2-one compound of the formula I 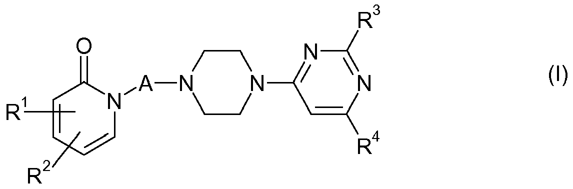 in which
A is a 4- to 6-membered hydrocarbon chain which may have 1 or 2 methyl groups as substituents, in which 1 or 2 carbon atoms may be replaced by oxygen, a carbonyl group or sulfur, and in which the hydrocarbon chain may have a double bond or a triple bond;
R¹, R² are independently of one another hydrogen, CN, NO₂, halogen, OR⁵, NR⁶R⁷, C(O)NR⁶R⁷, O-C(O)NR⁶R⁷, SR⁸, SOR⁸, SO₂R⁸, SO₂NR⁶R⁷, COOR⁹, O-C(O)R¹⁰, COR¹⁰, C₁-C₆-alkyl, C₁-C₆-haloalkyl, C₂-C₆-alkenyl, C₂-C₆-alkynyl, C₂-C₆-haloalkenyl, C₃-C₆-cycloalkyl, 4- to 6-membered heterocyclyl having 1, 2 or 3 heteroatoms sectected from 0, S and N, which may have 1, 2 or 3 substituents which are selected independently of one another from C₁-C₄-alkyl, C₁-C₄-alkoxy, NR⁶R⁷, CN, OH, C₁-C₂-fluoroalkyl or halogen, phenyl which may have 1, 2 or 3 substituents which are selected independently of one another from C₁-C₄-alkyl, C₁-C₄-alkoxy, NR⁶R⁷, OH, CN, C₁-C₂-fluoroalkyl or halogen, C₁-C₆-alkyl which has a substituent which is selected from OR⁵, NR⁶R⁷, C(O)NR⁶R⁷, O-C(O)NR⁶R⁷, SR⁸, SOR⁸, SO₂R⁸, SO₂NR⁶R⁷, COOR⁹, O-C(O)R¹⁰, COR¹⁰, C₃-C₆-cycloalkyl, 5- or 6-membered heterocyclyl having 1, 2 or 3 heteroatoms selected from 0, S and N, and phenyl, where phenyl and heterocyclyl may have 1, 2 or 3 substituents which are selected independently of one another from C₁-C₄-alkyl, C₁-C₄-alkoxy, NR⁶R⁷, CN, OH, C₁-C₂-fluoroalkyl or halogen, C₂-C₆-alkenyl which has a substituent selected from OR⁵, NR⁶R⁷, C(O)NR⁶R⁷, O-C(O)NR⁶R⁷, SR⁸, SOR⁸, SO₂R⁸, SO₂NR⁶R⁷, COOR⁹, O-C(O)R¹⁰, COR¹⁰, C₃-C₆-cycloalkyl, 5- or 6-membered heterocyclyl having 1, 2 or 3 heteroatoms selected from 0, S and N, and phenyl, where phenyl and heterocyclyl in turn may have 1, 2 or 3 substituents which are selected independently of one another from C₁-C₄-alkyl, C₁-C₄-alkoxy, NR⁶R⁷, OH, CN, C₁-C₂-fluoroalkyl or halogen;
R³, R⁴ are independently of one another OR⁵, NR⁶R⁷, CN, C₁-C₆-alkyl which is optionally substituted one or more times by OH, C₁-C₄-alkoxy, halogen or phenyl which in turn may have 1, 2 or 3 substituents selected from C₁-C₄-alkyl, C₁-C₄-alkoxy, NR⁶R⁷, OH, CN, C₁-C₂-fluoroalkyl or halogen, or C₂-C₆-alkenyl, C₂-C₆-alkynyl, C₃-C₆-cycloalkyl, C₄-C₁₀-bicycloalkyl, C₆-C₁₀-tricycloalkyl, where the last 5 groups mentioned may optionally be substituted one or more times by halogen or C₁-C₄-alkyl, or halogen, CN, C₁-C₄-alkoxy, 5- or 6-membered heterocyclyl having 1, 2 or 3 heteroatoms selected from 0, S and N, and phenyl, where phenyl and heterocyclyl may optionally have 1, 2 or 3 substituents which are selected independently of one another from C₁-C₄-alkyl, C₁-C₄-alkoxy, NR⁶R⁷, CN, C₁-C₂-fluoroalkyl and halogen;
R⁵, R⁶, R⁷ R⁸, R⁹ and R¹⁰ are independently of one another H, C₁-C₆-alkyl which is optionally substituted by OH, C₁-C₄-alkoxy or phenyl which in turn may have 1, 2 or 3 substituents selected from C₁-C₄-alkyl, C₁-C₄-alkoxy, NR⁶R⁷, OH, CN, C₁-C₂-fluoroalkyl or halogen, or C₁-C₆-haloalkyl or phenyl which in turn may have 1, 2 or 3 substituents selected from C₁-C₄-alkyl, C₁-C₄-alkoxy, NR⁶R⁷, OH, CN, C₁-C₂-fluoroalkyl or halogen, where
R⁷ may also be a COR¹¹ group, and where
R⁶ with R⁷ may also, together with the nitrogen to which they are bonded, form a 4-, 5- or 6-membered, saturated or unsaturated heterocycle which may have a further heteroatom selected from 0, S and NR¹² as ring member, where R¹² is hydrogen or C₁-C₄-alkyl, and which may be substituted by 1, 2, 3 or 4 alkyl groups; and
R¹¹ is hydrogen, C₁-C₄-alkyl or phenyl which is optionally substituted by 1, 2 or 3 radicals which are selected independently of one another from C₁-C₄-alkyl, C₁-C₄-alkoxy, NR⁶R⁷, CN, C₁-C₂-fluoroalkyl or halogen;
and the tautomers of the compound I, the physiologically acceptable salts of the compound I and the physiologically acceptable salts of the tautomers of the compound I.

2. A pyridin-2-one compound according to claim 1, in which R³ is C₁-C₆-alkyl, and R⁴ is selected from C₁-C₆-alkyl, C₃-C₆-cycloalkyl which optionally has 1 or 2 substituents selected from chlorine and methyl, and C₁-C₂-fluoroalkyl.

3. A pyridin-2-one compound according to claim 2, in which R³ is branched alkyl having 3, 4 or 5 C atoms or is C₃-C₆-cycloalkyl.

4. A pyridin-2-one compound according to claim 2 or 3, in which R⁴ is trifluoromethyl or C₃-C₄-alkyl.

5. A pyridin-2-one compound according to claim 2 or 3, in which R⁴ is cyclopropyl, cyclobutyl, cyclopentyl or 1-methylcyclopropyl.

6. A pyridin-2-one compound according to any of the preceding claims, in which at least one of the radicals R¹ or R² is different from hydrogen.

7. A pyridin-2-one compound according to claim 6, in which R¹ is selected from halogen, OR⁵, NR⁶R⁷, C₁-C₄-alkyl which is optionally substituted by OH, C₁-C₄-alkoxy or halogen, or aromatic 5- or 6-membered heterocyclyl having 1, 2 or 3 heteroatoms selected from 0, S and N, which may have 1, 2 or 3 substituents which are selected independently of one another from C₁-C₄-alkyl, C₁-C₄-alkoxy, NR⁶R⁷, CN, OH, C₁-C₂-fluoroalkyl or halogen,
and phenyl which may have 1, 2 or 3 substituents which are selected independently of one another from C₁-C₄-alkyl, C₁-C₄-alkoxy, NR⁶R⁷, OH, CN, C₁-C₂-fluoroalkyl or halogen.

8. A pyridin-2-one compound according to claim 7, in which R¹ is selected from phenyl, OH, chlorine, methyl, methoxy and trifluoromethyl.

9. A pyridin-2-one compound according to any of the preceding claims, in which R² is hydrogen.

10. A pyridin-2-one compound according to any of the preceding claims, in which A is butane-1,4-diyl.

11. A pharmaceutical composition comprising at least one compound according to any of claims 1 to 10 and/or salt thereof, optionally together with physiologically acceptable carriers and/or excipients.

12. The use of compounds according to any of claims 1 to 10 and of their pharmacologically acceptable salts for producing a pharmaceutical composition for the treatment of disorders which respond to influencing by dopamine D₃ receptor ligands.

13. The use of compounds according to any of claims 1 to 10 and of their pharmacologically acceptable salts for producing a pharmaceutical composition for the treatment of disorders of the central nervous system.

14. The use according to claim 13 for the treatment of schizophrenia and/or depression.

## Revendications

1. Composés de pyridin-2-one de formule générale I 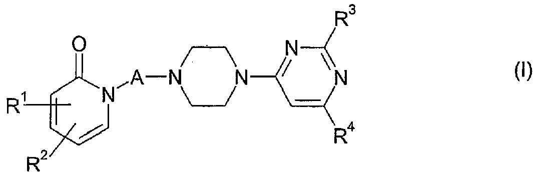 dans laquelle
A représente une chaîne hydrocarbonée de 4 à 6 chaînons, qui peut présenter 1 ou 2 groupes méthyle comme substituants, dans laquelle 1 ou 2 atomes de carbone peuvent être remplacés par de l'oxygène, un groupe carbonyle ou du soufre et dans laquelle la chaîne hydrocarbonée peut présenter une double liaison ou une triple liaison ;
R¹, R² représentent, indépendamment l'un de l'autre, hydrogène, CN, NO₂, halogène, OR⁵, NR⁶R⁷, C(O)NR⁶R⁷, O-C(O)NR⁶R⁷, SR⁸, SOR⁸, SO₂R⁸, SO₂NR⁶R⁷, COOR⁹, OC(O)R¹⁰, COR¹⁰, C₁-C₆-alkyle, C₁-C₆-halogénoalkyle, C₂-C₆-alcényle, C₂-C₆-alcynyle, C₂-C₆-halogénoalcényle, C₃-C₆-cycloalkyle, hétérocyclyle de 4 à 6 chaînons, comprenant 1, 2 ou 3 hétéroatomes, choisis parmi 0, S et N, qui peut présenter 1, 2 ou 3 substituants, qui sont choisis, indépendamment les uns des autres, parmi C₁-C₄-alkyle, C₁-C₄-alcoxy, NR⁶R⁷, CN, OH, C₁-C₂-fluoroalkyle ou halogène, phényle, qui peut présenter 1, 2 ou 3 substituants, qui sont choisis, indépendamment les uns des autres, parmi C₁-C₄-alkyle, C₁-C₄-alcoxy, NR⁶R⁷, CN, OH, C₁-C₂-fluoroalkyle ou halogène, C₁-C₆-alkyle, qui présente un substituant qui est choisi parmi OR⁵, NR⁶R⁷, C(O)NR⁶R⁷, O-C (0) NR⁶R⁷, SR⁸, SOR⁸, SO₂R⁸, SO₂NR⁶R⁷, COOR⁹, O-C(O)R¹⁰, COR¹⁰, C₃-C₆-cycloalkyle ; hétérocyclyle de 5 ou 6 chaînons comprenant 1, 2 ou 3 hétéroatomes, choisis parmi 0, S et N ; et phényle ; phényle et hétérocyclyle pouvant présenter 1, 2 ou 3 substituants qui sont choisis indépendamment les uns des autres parmi C₁-C₄-alkyle, C₁-C₄-alcoxy, NR⁶R⁷, CN, OH, C₁-C₂-fluoroalkyle ou halogène, C₂-C₆-alcényle, qui présente un substituant, choisi parmi OR⁵, NR⁶R⁷, C(O)NR⁶R⁷, O-C(O)NR⁶R⁷, SR⁸, SOR⁸, SO₂R⁸, SO₂NR⁶R⁷, COOR⁹, O-C (O) R¹⁰, COR¹⁰, C₃-C₆-cycloalkyle ; hétérocyclyle de 5 ou 6 chaînons comprenant 1, 2 ou 3 hétéroatomes, choisis parmi 0, S et N ; et phényle ; phényle et hétérocyclyle pouvant présenter à leur tour 1, 2 ou 3 substituants qui sont choisis indépendamment les uns des autres parmi C₁-C₄-alkyle, C₁-C₄-alcoxy, NR⁶R⁷, OH, CN, C₁-C₂-fluoroalkyle ou halogène ;
R³, R⁴ représentent, indépendamment l'un de l'autre, OR⁵, NR⁶R⁷, CN ; C₁-C₆-alkyle, qui est le cas échéant monosubstitué ou polysubstitué par OH, C₁-C₄-alcoxy, halogène ou phényle, qui peut à son tour porter 1, 2 ou 3 substituants choisis parmi C₁-C₄-alkyle, C₁-C₄-alcoxy, NR⁶R⁷, OH, CN, C₁-C₂-fluoroalkyle ou halogène ; C₂-C₆-alcényle, C₂-C₆-alcynyle, C₃-C₆-cycloalkyle, C₄-C₁₀-bicycloalkyle, C₆-C₁₀-tricycloalkyle, les 5 derniers groupes mentionnés pouvant le cas échéant être monosubstitués ou polysubstitués par halogène ou C₁-C₄-alkyle ; halogène, CN, C₁-C₄-alcoxy, hétérocyclyle de 5 ou 6 chaînons comprenant 1, 2 ou 3 hétéroatomes, choisis parmi 0, S et N ; et phényle ; phényle et hétérocyclyle portant le cas échéant 1, 2 ou 3 substituants, qui sont choisis, indépendamment les uns des autres, parmi C₁-C₄-alkyle, C₁-C₄-alcoxy, NR⁶R⁷, CN, C₁-C₂-fluoroalkyle et halogène ;
R⁵, R⁶, R⁷ R⁸, R⁹ et R¹⁰ représentent, indépendamment les uns des autres, H ; C₁-C₆-alkyle, qui est le cas échéant substitué par OH, C₁-C₄-alcoxy ou phényle, qui peut porter à son tour 1, 2 ou 3 substituants, choisis parmi C₁-C₄-alkyle, C₁-C₄-alcoxy, NR⁶R⁷, OH, CN, C₁-C₂-fluoroalkyle ou halogène ; C₁-C₆-halogénoalkyle ou phényle, qui peut porter à son tour 1, 2 ou 3 substituants, choisis parmi C₁-C₄-alkyle, C₁-C₄-alcoxy, NR⁶R⁷, OH, CN, C₁-C₂-fluoroalkyle ou halogène,
R⁷ pouvant également signifier un groupe COR¹¹, et
R⁶ pouvant également former avec R⁷, ensemble avec l'atome d'azote auquel ils sont liés, un hétérocycle saturé ou insaturé de 4, 5 ou 6 chaînons, qui peut présenter un autre hétéroatome, choisi parmi 0, S et NR¹² comme élément de cycle, R¹² pouvant représenter hydrogène ou C₁-C₄-alkyle, et qui peut être substitué par 1, 2, 3 ou 4 groupes alkyle ; et
R¹¹ représente hydrogène, C₁-C₄-alkyle ou phényle, qui est le cas échéant substitué par 1, 2 ou 3 radicaux, qui sont choisis indépendamment les uns des autres parmi C₁-C₄-alkyle, C₁-C₄-alcoxy, NR⁶R⁷, CN, C₁-C₂-fluoroalkyle ou halogène ;
ainsi que les tautomères des composés I, les sels physiologiquement acceptables des composés I et les sels physiologiquement acceptables des tautomères des composés I.

2. Composés de pyridin-2-one selon la revendication 1, dans lesquels R³ représente C₁-C₆-alkyle et R⁴ est choisi parmi C₁-C₆-alkyle, C₃-C₆-cycloalkyle, qui présente le cas échéant 1 ou 2 substituants, choisis parmi chlore et méthyle ; et C₁-C₂-fluoroalkyle.

3. Composés de pyridin-2-one selon la revendication 2, dans lesquels R³ représente alkyle ramifié comprenant 3, 4 ou 5 atomes de carbone ou C₃-C₆-cycloalkyle.

4. Composés de pyridin-2-one selon la revendication 2 ou 3, dans lesquels R⁴ représente trifluorométhyle ou C₃-C₄-alkyle.

5. Composés de pyridin-2-one selon la revendication 2 ou 3, dans lesquels R⁴ représente cyclopropyle, cyclobutyle, cyclopentyle ou 1-méthylcyclopropyle.

6. Composés de pyridin-2-one selon l'une quelconque des revendications précédentes, au moins un des radicaux R¹ ou R² étant différent d'hydrogène.

7. Composés de pyridin-2-one selon la revendication 6, dans lesquels R¹ est choisi parmi halogène, OR⁵, NR⁶R⁷, C₁-C₄-alkyle, qui est le cas échéant substitué par OH, C₁-C₄-alcoxy ou halogène ; hétérocyclyle aromatique de 5 ou 6 chaînons, comprenant 1, 2 ou 3 hétéroatomes, choisis parmi 0, S et N, qui peut présenter 1, 2 ou 3 substituants choisis indépendamment les uns des autres parmi C₁-C₄-alkyle, C₁-C₄-alcoxy, NR⁶R⁷, CN, OH, C₁-C₂-fluoroalkyle ou halogène,
et phényle, qui peut présenter 1, 2 ou 3 substituants, qui sont choisis, indépendamment les uns des autres, parmi C₁-C₄-alkyle, C₁-C₄-alcoxy, NR⁶R⁷, OH, CN, C₁-C₂-fluoroalkyle ou halogène.

8. Composés de pyridin-2-one selon la revendication 7, dans lesquels R¹ est choisi parmi phényle, OH, chlore, méthyle, méthoxy et trifluorométhyle.

9. Composés de pyridin-2-one selon l'une quelconque des revendications précédentes, dans lesquels R² signifie hydrogène.

10. Composés de pyridin-2-one selon l'une quelconque des revendications précédentes, dans lesquels A représente butane-1,4-diyle.

11. Agent pharmaceutique, contenant au moins un composé selon l'une quelconque des revendications 1 à 10 et/ou son sel, le cas échéant ensemble avec des supports et/ou des adjuvants physiologiquement acceptables.

12. Utilisation de composés selon l'une quelconque des revendications 1 à 10 et de leurs sels pharmacologiquement acceptables pour la préparation d'un agent pharmaceutique destiné au traitement de maladies qui répondent à l'influence par des ligands des récepteurs dopamine-D₃.

13. Utilisation de composés selon l'une quelconque des revendications 1 à 10 et de leurs sels pharmacologiquement acceptables pour la préparation d'un agent pharmaceutique destiné au traitement de maladies du système nerveux central.

14. Utilisation selon la revendication 13 pour le traitement de la schizophrénie et/ou de la dépression.
